# EUROPEAN PATENT SPECIFICATION

(11) **EP 1 051 397 B1**
(45) Date of publication and mention of the grant of the patent: **31.12.2008**
(21) Application number: 99904421.7
(22) Date of filing: 29.01.1999
(51) Int. Cl.: C07D 205/00, C07D 205/08, C07D 243/12, C07D 243/24, C07D 241/04, C07D 241/36, C07D 231/00, C07D 233/22, C07D 207/00, C07D 207/12, C07D 401/00, C07D 241/08

(54) **METHOD FOR PREPARING AN N- (ALIPHATIC OR AROMATIC)CARBONYL -2-AMINOACETAMIDE COMPOUND AND A CYCLYZED COMPOUND**
VERFAHREN ZUR HERSTELLUNG EINER N-¬(ALIPHATHISCH ODER AROMATISCH)CARBONYL -2-AMINOACETAMID-VERBINDUNG UND EINER CYCLISCHEN VERBINDUNG
PROCEDE DE PREPARATION D'UN COMPOSE N- CARBONYL(ALIPHATIQUE OU AROMATIQUE) -2-AMINOACETAMIDE ET D'UN COMPOSE CYCLISE

(30) Priority: 29.01.1998 US 73007 P; 31.08.1998 US 98404 P; 01.09.1998 US 98708 P; 18.09.1998 US 101056 P
(43) Date of publication of application: 15.11.2000
(73) Proprietor: Aventis Pharmaceuticals Inc., Bridgewater, NJ 08807 (US)
(72) Inventor: HULME, Christopher, Phoenixville, PA 19460 (US); MORTON, George, C., Collegeville, PA 19426 (US); SALVINO, Joseph, M., Schwenksville, PA 19473 (US); LABAUDINIERE, Richard, F., Collegeville, PA 19426 (US); MASON, Helen, J., Skillman, NJ 08558 (US); MORRISSETTE, Matthew, M., Pottstown, PA 19464 (US); MA, Liang, King of Prussia, PA 19406 (US); CHERRIER, Marie-Pierre, Phoenixville, PA 19460 (US)
(74) Representative: Jones, Stephen Anthony
(86) International application number: PCT/US1999/001923
(87) International publication number: WO 1999/038844

(56) References cited:
- WO-A-97/48685
- TH. A. KEATING: "a remarkable two-step synthesis of diverse 1,4-benzodiazepine-2,5-diones using the ugi four-component condensation." JOURNAL OF ORGANIC CHEMISTRY., vol. 61, no. 25, 1996, pages 8935-9, XP002290623 USAMERICAN CHEMICAL SOCIETY. EASTON.
- FUKUYAMA et al., "Synthetic Approach to Bicyclomycin: Synthesis of the Bicyclic System of Bicyclomycin", TETRAHEDRON LETTERS, 1981, Vol. 22, No. 42, pages 4155-4158, XP002920814
- FAILLI et al., "Model Experiments Directed Towards the Synthesis of N-Aminopeptides", CANADIAN JOURNAL OF CHEMISTRY, 1973, Vol. 51, pages 2769-2775, XP002920815
- BOEHM et al., "Rapid and Convenient Synthesis of Polyoxin Containing N-Methylated Peptide Bonds", JOURNAL OF ORGANIC CHEMISTRY, 1986, Vol. 51, pages 2307-2314, XP002920816

## Description

This invention is directed to a method for preparing an N-[(aliphatic or aromatic)carbonyl)]-2-aminoacetamide compound, and a cyclized compound therefrom, and the resin bound isonitrile used in their preparation.

### Background of the Invention

1,4-benzodiazepine-2,5-diones are an important class of biologically actives compounds. This class of compounds has been identified as having platelet aggregation inhibitor activity, anticonvulsant activity, anxiolytic activity and as anti tumor agents (Mc Dowell, R.S. et al., J. Am. Chem. Soc., 1994, 116, 5077; Cho, N.S. et al., J Heterocycl. Chem., 1989, 26, 1807; Wright, W.B. et al., J. Med. Chem., 1978, 21, 1087; Jones; G.B. et al., Anti-Cancer Drug Des. 1990, 5, 249).

Benzodiazepines have been to shown to have utility as GPIIb/IIIa receptor antagonists (Ku, T. W.; Miller, W. H.; Bondinell, W. E.; Erhard, K. F.; Keenan, R. M.; Nichols, A. J.; Peishoff, C. E.; Samenen, J. M.; Wong, A. S.; Huffman, W. F. J. Med. Chem. 1995, 38, 9 - 12) and may be useful for the treatment of acute myocardial infarction, unstable angina, or thrombotic stroke. Recent developments have extended the therapeutic utility of this class of molecule to include integrin antagonists (for example antagonists of the vitronectin receptor), useful for the stimulation of bone formation and treatment of bone fractures, osteoporosis and other bone-related disorders (Drake, F.H. WO98115278-A1, 1997).

Pressures on the pharmaceutical industry have increased significantly to meet the economic challenges of the 1990s. As a consequence, efforts in both industrial and academic sectors are now being directed at new technologies for attacking drug discovery in a more efficient and cost-effective manner. As such, with the recent development of combinatorial chemistry and high speed parallel synthesis within the Lead Discovery arena, the multi-component reaction (MCR) has witnessed a resurgence of interest. From a practical consideration one-pot reactions, such as the Ugi and Passerini reactions, are easily automated and production of diverse or directed libraries of small organic molecules is thus both facile and high-throughput. Despite this tremendous synthetic potential, the Ugi reaction is limited by producing products that are flexible and peptidic-like, often being classified as 'non-drug-like' and suffering from bioavailability problems. Interestingly several novel intramolecular derivatives of this versatile reaction have recently been reported where constrained products are achieved by intercepting the intermediate nitrilium ion of the Ugi reaction. An alternative approach and the one described in this application is to constrain the Ugi product via a so-called secondary reaction after initial formation of the classical Ugi product. Production of the derivatives described herein is facile and amenable to automated high throughput production, allowing production of vast arrays of biologically relevant molecules (in the range of at least 10,000 molecules/template revealed in good purity).

Keating, Th. A. J. Org. Chem. 1996, 61(25), 8935-9 describes a two-step, general synthesis of 1,4-benzodiazepine-2,5-diones that employs an Ugi four-component condensation using a convertible isocyanide (1-isocyanocylohexene).

### Summary of the Invention

The present invention relates to a method for preparing an N-[(aliphatic or aromatic)carbonyl)]-2-aminoacetamide compound of the formula 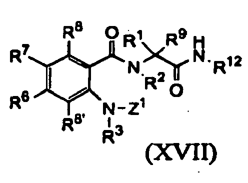 comprising
reacting the following four compounds:
a carbonyl compound of formula 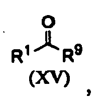
an amine compound of formula

   R²-NH₂ (XVI),
an isonitrile compound of formula

   R¹²-NC (IX),

   and
an acid compound of formula 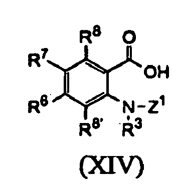 wherein Z¹ is a suitable amine protecting group,
to produce the N-[(aliphatic or aromatic)carbonyl)]-2-aminoacetamide compound;
wherein:
R¹ and R⁹ independently are selected from hydrogen, alkenyl, alkyl, aralkenyl, aralkyl, aryl, fused arylcycloalkenyl, fused arylcycloalkyl, fused arylheterocyclenyl, fused arylheterocyclyl, cycloalkyl, cycloalkenyl, heteroaralkenyl, heteroaralkyl, heteroaryl, fused heteroarylcycloalkenyl, fused heteroarylcycloalkyl, fused heteroarylheterocyclenyl, fused heteroarylheterocyclyl, heterocyclenyl, and heterocyclyl;
R² is selected from hydrogen, alkenyl, alkyl, aralkyl, aryl, fused arylcycloalkenyl, fused arylcycloalkyl, fused arylheterocyclenyl, fused arylheterocyclyl, cycloalkyl, cycloalkenyl, heteroaralkyl, heteroaryl, fused heteroarylcycloalkenyl, fused heteroarylcycloalkyl, fused heteroarylheterocyclenyl, fused heteroarylheterocyclyl, heterocyclenyl and heterocyclyl;
R³ is selected from hydrogen, alkenyl, alkyl, aralkyl, aryl, fused arylcycloalkenyl, fused arylcycloalkyl, fused arylheterocyclenyl, fused arylheterocyclyl, cycloalkyl, cycloalkenyl, heteroaralkyl, heteroaryl, fused heteroarylcycloalkenyl, fused heteroarylcycloalkyl, fused heteroarylheterocyclenyl, fused heteroarylheterocyclyl, heterocyclenyl and heterocyclyl;
R⁶, R⁷, R⁸ and R^{8'} independently are selected from hydrogen, alkenyl, alkenyloxy, alkoxy, alkyl, aryl, alkylsulfinylcarbamoyl, alkynyl, alkynyloxy, aralkenyl, aralkylsulfonyl, aralkynyl, fused arylcycloalkenyl, fused arylcycloalkyl, fused arylheterocyclenyl, fused arylheterocyclyl, aryloxycarbonyl, cycloalkyloxy, heteroaralkenyl, heteroaralkyloxy; heteroaralkynyl, heteroaroyl, fused heteroarylcycloalkenyl, fused heteroarylcycloalkyl, fused heteroarylheterocyclenyl, fused heteroarylheterocyclyl, heteroarylsulphonylcarbamoyl, heterocyclyloxy, heteroaryl, aralkyl, heteroaralkyl, hydroxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, aryldiazo, heteroaryldiazo, amidino, Y¹Y²N-, Y¹Y²NCO- and Y¹Y²NSO₂-, wherein Y¹ and Y² are independently hydrogen, alkyl, aryl, aralkyl or heteroaralkyl, or where the substituent is Y¹Y²N-, then one of Y¹ and Y² may be acyl or aroyl and the other of Y¹ and Y² is as defined previously, or where the substituent is Y¹Y²NCO- or Y¹Y²NSO₂-, Y¹ and Y², taken together with the N atom through which Y¹ and Y² are linked, may also form a 4 to 7 membered heterocyclyl or heterocyclenyl, or
R³ and R^{8'}, taken together with the nitrogen atom and carbon atoms through which R³ and R^{8'} are linked, form a 5 to 7 membered heterocyclyl or heterocyclenyl, or
two adjacent substituents selected from the substituents R⁶, R⁷, R^{8'} and R⁸, taken together with the aryl carbon atoms through which the two adjacent substituents are linked, form a 5 to 7 membered cycloalkyl or a cycloalkenyl, heterocyclyl or heterocyclenyl, or 6 membered aryl or 5 or 6 membered heteroaryl ring; and
wherein
the isonitrile compound (IX) is selected from the group of formulae 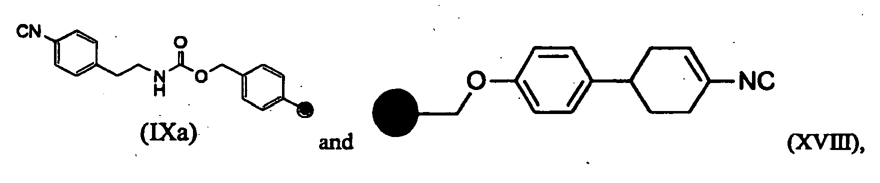 where 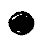 is a solid support resin.

### DETAILED DESCRIPTION

As used above, and throughout the description of the invention, the following terms, unless otherwise indicated, shall be understood to have the following meanings:-
"Acid bioisostere" means a group which has chemical and physical similarities producing broadly similar biological properties to a carboxy group (see Lipinski, Annual Reports in Medicinal Chemistry, 1986,21, p.283 "Bioisosterism In Drug Design"; Yun, Hwahak Sekye, 1993, 33, p.576-579 "Application Of Bioisosterism To New Drug Design"; Zhao, Huaxue Tongbao, 1995, p.34-38 "Bioisosteric Replacement And Development Of Lead Compounds In Drug Design"; Graham, Theochem, 1995, 343, p.105-109 "Theoretical Studies Applied To Drug Design:ab initio Electronic Distributions In Bioisosteres"). Examples of suitable acid bioisosteres include: -C(=O)-NHOH, -C(=O)-CH₂OH, -C(=O)-CH₂SH, -C(=O)-NH-CN, sulpho, phosphono, alkylsulphonylcarbamoyl, tetrazolyl, arylsulphonylcarbamoyl, heteroarylsulphonylcarbamoyl, N-methoxycarbamoyl, 3-hydroxy-3-cyclobutene-1,2-dione, 3,5-dioxo-1,2,4-oxadiazolidinyl or heterocyclic phenols such as 3-hydroxyisoxazolyl and 3-hydoxy-1-methylpyrazolyl.
"Acidic functional group" means a group with an acidic hydrogen within it. The "corresponding protected derivatives" are those where the acidic hydrogen atom has been replaced with a suitable protecting group, to block or protect the acid functionality while the reactions involving other functional sites of the compound are carried out. Such protecting groups are well known to those skilled in the art, having been extensively used in the protection of carboxyl groups in the penicillin and cephalosporin fields, as described in U.S. Pat. No. 3,840,556 and 3,719,667, the disclosures of which are hereby incorporated herein by reference. For suitable protecting groups see T.W. Green and P.G.M.Wuts in "Protective Groups in Organic Chemistry" John Wiley and Sons, 1991. Exemplary acidic functional groups include carboxyl (and acid bioisosteres), hydroxy, mercapto and imidazole. Examples of carboxylic acid protecting groups include esters such as methoxymethyl, methylthiomethyl, tetrahydropyranyl, substituted and unsubstituted phenacyl, 2,2,2-trichlomethyl, *tert*-butyl, cinnamyl, dialkylaminoalkyl (e.g., dimethylaminoethyl and the like), trimethylsilyl, and the like, and amides and hydrazides including N,N-dimethyl, 7-nitroindolyl, hydrazide, N-phenylhydrazide, C₁ to C₈ loweralkyl (e.g., methyl, ethyl or tertiary butyl and the like); and substituted derivatives thereof such as alkoxybenzyl or nitrobenzyl groups and the like; alkanoyloxyalkyl groups such as pivaloyloxymethyl or propionyloxymethyl and the like; aroyloxyalkyl, such as benzoyloxyethyl and the like; alkoxycarbonylalkyl, such as methoxycarbonylmethyl, cyclohexyloxycarbonylmethyl and the like; alkoxycarbonyloxyalkyl, such as t-butyloxycarbonyloxymethyl and the like; alkoxycarbonylaminoalkyl, such as t-butyloxycarbonylaminomethyl and the like; alkylaminocarbonylaminoalkyl, such as methylaminocarbonylaminomethyl and the like; alkanoylaminoalkyl, such as acetylaminomethyl and the like; heterocycliccarbonyloxyalkyl, such as 4-methylpiperazinylcarbonyloxymethyl and the like; dialkylaminocarbonylalkyl, such as dimethylaminocarbonylmethyl and the like; (5-(loweralkyl)-2-oxo-1,3-dioxolen-4-yl)alkyl, such as (5-t-butyl-2-oxo-1,3-dioxolen-4-yl)methyl and the like; and (5-phenyl-2-oxo-1,3-dioxolen-4-yl)alkyl, such as (5-phenyl-2-oxo-l,3-dioxolen-4-yl)methyl and the like.
"Acyl" means an H-CO- or alkyl-CO- group wherein the alkyl group is as herein described. Preferred acyls contain a lower alkyl. Exemplary acyl groups include formyl, acetyl, propanoyl, 2-methylpropanoyl, t-butylacetyl, butanoyl and palmitoyl.
"Aliphatic" means a radical derived from a non aromatic C-H bond by removal of the hydrogen atom. The aliphatic radical may be further substituted by additional aliphatic or aromatic radicals as defined herein. Representative aliphatic groups include alkyl, alkenyl, alkynyl, cycloalkyl, cycloalkenyl, heterocyclyl, heterocyclenyl, aralkenyl, aralkyloxyalkyl, aralkyloxycarbonylalkyl, aralkyl, aralkynyl, aralkyloxyalkenyl, heteroaralkenyl, heteroaralkyl, heteroaralkyloxyalkenyl, heteroaralkyloxyalkyl, heteroaralkynyl, fused arylcycloalkyl, fused heteroarylcycloalkyl, fused arylcycloalkenyl, fused heteroarylcycloalkenyl, fused arylheterocyclyl, fused heteroarylheterocyclyl, fused arylheterocyclenyl, fused heteroarylheterocyclenyl, and the like. "Aliphatic", as used herein, also encompasses the residual, non-carboxyl portion of natural and unnatural amino acids as defined herein.
"Aromatic" means a radical derived from an aromatic C-H bond by removal of the hydrogen atom. Aromatic includes both aryl and heteroaryl rings as defined herein. The aryl or heteroaryl ring may be further substituted by additional aliphatic or aromatic radicals as defined herein. Representative aromatic groups include aryl, fused cycloalkenylaryl, fused cycloalkylaryl, fused heterocyclylaryl, fused heterocyclenylaryl, heteroaryl, fused cycloalkylheteroaryl, fused cycloalkenylheteroaryl, fused heterocyclenylheteroaryl, fused heterocyclylheteroaryl, and the like.
"Acylamino" is an acyl-NH- group wherein acyl is as defined herein.
"Alkenoyl" means an alkenyl-CO- group wherein alkenyl is as defined herein.
"Alkenyl" means an aliphatic hydrocarbon group containing a carbon-carbon double bond and which may be straight or branched having about 2 to about 15 carbon atoms in the chain. Preferred alkenyl groups have 2 to about 12 carbon atoms in the chain; and more preferably about 2 to about 5 carbon atoms in the chain. Branched means that one or more lower alkyl groups such as methyl, ethyl or propyl are attached to a linear alkenyl chain. "Lower alkenyl" means about 2 to about 4 carbon atoms in the chain which may be straight or branched. The alkenyl group may be substituted with one or more "alkenyl group substituents" which may be the same or different, and include halo, alkenyloxy, cycloalkyl, cyano, hydroxy, alkoxy, carboxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkyloxycarbonyl or Y¹Y²N-, Y¹Y²NCO- or Y¹Y²NSO₂-, wherein Y¹ and Y² are independently hydrogen, alkyl, aryl, aralkyl or heteroaralkyl, or for where the substituent is Y¹Y²N-, then one of Y¹ and Y² may be acyl or aroyl as defined herein and the other of Y¹ and Y² is as defined previously, or for where the substituent is Y¹Y²NCO- or Y¹Y²NSO₂, Y¹ and Y² may also be taken together with the N atom through which Y¹ and Y² are linked to form a 4 to 7 membered heterocyclyl or heterocyclenyl. Exemplary alkyl groups include methyl, trifluoromethyl, cyclopropylmethyl, cyclopentylmethyl, ethyl, *n*-propyl, *i*-propyl, *n*-butyl, *t-*butyl, *n*-pentyl, 3-pentyl, methoxyethyl, carboxymethyl, methoxycarbonylethyl, benzyloxycarbonylmethyl, and pyridylmethyloxycarbonylmethyl. Exemplary alkenyl groups include ethenyl, propenyl, *n*-butenyl, *i*-butenyl, 3-methylbut-2-enyl, *n*-pentenyl, heptenyl, octenyl, cyclohexylbutenyl and decenyl.
"Alkenyloxy" means an alkenyl-O- group wherein the alkenyl group is as herein described. Exemplary alkenyloxy groups include allyloxy and 3-butenyloxy.
"Alkenyloxyalkyl" means alkenyl-O-alkyl group wherein the alkyl and alkenyl groups are as described herein.
"Alkoxy" means an alkyl-O- group wherein the alkyl group is as herein described. Exemplary alkoxy groups include methoxy, ethoxy, n-propoxy, *i*-propoxy, n-butoxy and heptoxy.
"Alkoxyalkyl" means an alkyl-0-alkyl- group wherein the alkyl groups are independent as herein described. Exemplary alkoxy groups include methoxyethyl, ethoxymethyl, n-butoxymethyl and cyclopentylmethyloxyethyl.
"Aminoiminomethyl" means a NH₂C(=NH)- group. It is known that this moiety may be mono or di-protected to afford, for example (alkoxycarbonylamino)iminomethyl and (alkoxycarbonylamino)alkoxycarbonyliminomethyl groups.
"Alkoxycarbonyl" means an alkyl-O-CO- group, wherein the alkyl group is as herein defined. Exemplary alkoxycarbonyl groups include methoxycarbonyl, ethoxycarbonyl, and t-butyloxycarbonyl.
"Alkoxycarbonylalkyl" means an alkyl-O-OC-alkyl- group wherein the alkyl groups are as herein defined. Preferred groups include methoxy- and ethoxy-carbonylmethyl and carbonyl ethyl.
"Alkyl" means an aliphatic hydrocarbon group which may be straight or branched having about 1 to about 20 carbon atoms in the chain. Preferred alkyl groups have 1 to about 12 carbon atoms in the chain. Branched means that one or more lower alkyl groups such as methyl, ethyl or propyl are attached to a linear alkyl chain. "Lower alkyl" means about 1 to about 4 carbon atoms in the chain which may be straight or branched. The alkyl may be substituted with one or more "alkyl group substituents" which may be the same or different, and include halo, alkenyloxy, cycloalkyl, aroyl, cyano, hydroxy, alkoxy, carboxy, alkynyloxy, aralkoxy, aryloxy, aryloxycarbonyl, alkylthio, heteroarylthio, aralkylthio, arylsulphonyl, alkylsulphonyl, alkylphosphonate, heteroaralkyloxy, heterocyclyl, fused heteroarylcycloalkenyl, fused heteroarylcycloalkyl , fused heteroarylheterocyclenyl, fused heteroarylheterocyclyl, fused arylcycloalkenyl, fused arylcycloalkyl, fused arylheterocyclenyl, fused arylheterocyclyl, alkoxycarbonyl, aralkoxycarbonyl, (alkoxycarbonylamino)iminomethyl; (alkoxycarbonylamino)alkoxycarbonyliminomethyl, heteroaralkyloxycarbonyl or Y¹Y²N-, Y¹Y²NCO- or Y¹Y²NSO₂-, wherein Y¹ and Y² are independently hydrogen, alkyl, aryl, heteroaroyl, aralkyl or heteroaralkyl, or for where the substituent is Y¹Y²N-, then one of Y¹ and Y² may be acyl, alkoxycarbonyl or aroyl as defined herein and the other of Y¹ and Y² is as defined previously, or for where the substituent is Y¹Y²NCO- or Y¹Y²NSO₂, Y¹ and Y² may also be taken together with the N atom through which Y¹ and Y² are linked to form a 4 to 7 membered heterocyclyl or heterocyclenyl. Exemplary alkyl groups include methyl, trifluoromethyl, cyclopropylmethyl, cyclopentylmethyl, ethyl, *n*-propyl, *i*-propyl, *n*-butyl, *t*-butyl, *n*-pentyl, n-nonyl, decyl, 3-pentyl, methoxyethyl, carboxymethyl, methoxycarbonylethyl, benzyloxycarbonylmethyl, and pyridylmethyloxycarbonylmethyl. Preferred alkyl group substituents are fused arylcycloalkenyl, cyano, fused arylcycloalkyl, aralkylthio, Y¹Y²N-, Y¹Y²NCO-, fused arylheterocyclenyl, fused arylheterocyclyl, hydroxy, heterocyclyl, aralkoxy, alkoxycarbonyl, alkylthio, aryloxy, aroyl, heteroaroyl, arylsulphonyl, heteroarylthio alkylphosphonate, alkylsulphonyl, (alkoxycarbonylamino)iminomethyl, (alkoxycarbonylamino)alkoxycarbonyliminomethyl, and cycloalkyl.
"Alkylcarbamoyl" is an alkyl-NH-CO- group wherein the alkyl group is herein defined.
"Alkylphosphonate" means an (alkylO)₂P=O- group wherein the alkyl groups are independent of each other are herein defined.
"Alkylsulfinyl" means an alkyl-SO- group wherein the alkyl group is as defined above. Preferred groups are those wherein the alkyl group is lower alkyl.
"Alkylsulfonyl" means an alkyl-SO₂- group wherein the alkyl group is as defined above. Preferred groups are those wherein the alkyl group is lower alkyl.
"Alkylsulphonylcarbamoyl" means an alkyl-SO₂-NH-C(=O)- group wherein the alkyl group is as herein described. Preferred alkylsulphonylcarbamoyl groups are those wherein the alkyl group is C₁₋₄ alkyl.
"Alkylthio" means an alkyl-S- group wherein the alkyl group is as herein described. Exemplary alkylthio groups include methylthio, ethylthio, *i*-propylthio and heptylthio.
"Alkynyl" means an aliphatic hydrocarbon group containing a carbon-carbon triple bond and which may be straight or branched having about 2 to about 15 carbon atoms in the chain. Preferred alkynyl groups have 2 to about 12 carbon atoms in the chain; and more preferably about 2 to about 4 carbon atoms in the chain. Branched means that one or more lower alkyl groups such as methyl, ethyl or propyl are attached to a linear alkynyl chain. "Lower alkynyl" means about 2 to about 4 carbon atoms in the chain which may be straight or branched. The alkynyl group may be substituted with one or more "alkynyl group substituents" which may be the same or different, and include halo, alkenyloxy, cycloakyl, cyano, hydroxy, alkoxy, carboxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkyloxycarbonyl or Y¹Y²N-, Y¹Y²NCO- or Y¹Y²NSO₂-, wherein Y¹ and Y² are independently hydrogen, alkyl, aryl, aralkyl or heteroaralkyl, or for where the substituent is Y¹Y²N-, then one of Y¹ and Y² may be acyl or aroyl as defined herein and the other of Y¹ and Y² is as defined previously, or for where the substituent is Y¹Y²NCO- or Y¹Y²NSO₂, Y¹ and Y² may also be taken together with the N atom through which Y¹ and Y² are linked to form a 4 to 7 membered heterocyclyl or heterocyclenyl. Exemplary alkyl groups include methyl, trifluoromethyl, cyclopropylmethyl, cyclopentylmethyl, ethyl, *n*-propyl, *i*-propyl, *n*-butyl, *t-*butyl, *n*-pentyl, 3-pentyl, methoxyethyl, carboxymethyl, methoxycarbonylethyl, benzyloxycarbonylmethyl, pyridylmethyloxycarbonylmethyl. Exemplary alkynyl groups include ethynyl, propynyl, n-butynyl, 2-butynyl, 3-methylbutynyl, n-pentynyl, heptynyl, octynyl and decynyl.
"Alkynyloxy" means an alkynyl-O- group wherein the alkynyl group is as herein described. Exemplary alkynyloxy groups include propynyloxy and 3-butynyloxy.
"Amino acid" means an amino acid selected from the group consisting of natural and unnatural amino acids as defined herein. Preferred amino acids are those possessing an α-amino group. The amino acids may be neutral, positive or negative depending on the substituents in the side chain. "Neutral amino acid" means an amino acid containing uncharged side chain substituents. Exemplary neutral amino acids include alanine, valine, leucine, isoleucine, proline, phenylalanine, tryptophan, methionine, glycine, serine, threonine and cysteine. "Positive amino acid" means an amino acid in which the side chain substituents are positively charged at physiological pH. Exemplary positive amino acids include lysine, arginine and histidine. "Negative amino acid" means an amino acid in which the side chain substituents bear a net negative charge at physiological pH. Exemplary negative amino acids include aspartic acid and glutamic acid. Preferred amino acids are α-amino acids. Exemplary natural amino acids are isoleucine, proline, phenylalanine, tryptophan, methionine, glycine, serine, threonine, cysteine, tyrosine, asparagine, glutamine, lysine, arginine, histidine, aspartic acid and glutamic acid.
"Unnatural amino acid" means an amino acid for which there is no nucleic acid codon. Examples of unnatural amino acids include, for example, the D-isomers of the natural α-amino acids as indicated above; Aib (aminobutyric acid), βAib (3-aminoisobutyric acid), Nva (norvaline), β-Ala, Aad (2-aminoadipic acid), βAad (3-aminoadipic acid), Abu (2-aminobutyric acid), Gaba (γ-aminobutyric acid), Acp (6-aminocaproic acid), Dbu (2,4-diaminobutryic acid), α-aminopimelic acid, TMSA (trimethylsilyl-Ala), aIle (allo-isoleucine), Nle (norleucine), *tert*-Leu, Cit (citrulline), Orn, Dpm (2,2'-diaminopimelic acid), Dpr (2,3-diaminopropionic acid), α- or β-Nal, Cha (cyclohexyl-Ala), hydroxyproline, Sar (sarcosine), and the like; cyclic amino acids; N-α-alkylated amino acids such as MeGly (N-α-methylglycine), EtGly (N-α-ethylglycine) and EtAsn (N-α-ethylasparagine); and amino acids in which the α-carbon bears two side-chain substituents. The names of natural and unnatural amino acids and residues thereof used herein follow the naming conventions suggested by the IUPAC Commission on the Nomenclature of Organic Chemistry and the IUPAC-IUB Commission on Biochemical Nomenclature as set out in "Nomenclature of α-Amino Acids (Recommendations, 1974) " Biochemistry, 14(2), (1975). To the extent that the names and abbreviations of amino acids and residues thereof employed in this specification and appended claims differ from those noted, differing names and abbreviations will be made clear.
"Amino acid side chains" means the substituent found on the carbon between the amino and carboxy groups in α-amino acids. For examples of "corresponding protected derivatives" of amino acid side chains, see T.W. Green and P. G. M. Wuts in "Protective Groups in Organic Chemistry" John Wiley and Sons, 1991.
"Amine protecting group" means an easily removable group which is known in the art to protect an amino group against undesirable reaction during synthetic procedures and to be selectively removable. The use of amine protecting groups is well known in the art for protecting groups against undesirable reactions during a synthetic procedure and many such protecting groups are known, cf, for example, T.H. Greene and P.G.M. Wuts, Protective Groups in Organic Synthesis, 2nd edition, John Wiley & Sons, New York (1991), incorporated herein by reference. Preferred amine protecting groups are acyl, including formyl, acetyl, chloroacetyl, trichloroacetyl, o-nitrophenylacetyl, o-nitrophenoxyacetyl, trifluoroacetyl, acetoacetyl, 4-chlorobutyryl, isobutyryl, o-nitrocinnamoyl, picolinoyl, acylisothiocyanate, aminocaproyl, benzoyl and the like, and acyloxy including methoxycarbonyl, 9-fluorenylmethoxycarbonyl, 2,2,2-trifluoroethoxycarbonyl, 2-trimethylsilylethxoycarbonyl, vinyloxycarbonyl, allyloxycarbonyl, *t*-butyloxycarbonyl (BOC), 1,1-dimethylpropynyloxycarbonyl, benzyloxycarbonyl (CBZ), p-nitrobenzyloxycarbony, 2,4-dichlorobenzyloxycarbonyl, and the like.
"Acid labile amine protecting group" means an amine protecting group as defined above which is readily removed by treatment with acid while remaining relatively stable to other reagents. Preferred acid labile amine protecting groups include *tert*-butoxycarbonyl (BOC), 2-(4-biphenyl)-isopropoxy carbonyl.
"Base labile amine protecting group" means an amine protecting group as defined above which is readily removed by treatment with base while remaining relatively stable to other reagents. Preferred base labile amine protecting groups include 9-fluoroenylmethoxycarbonyl (FMOC).
"Hydrogenation labile amine protecting group" means an amine protecting group as defined above which is readily removed by hydrogenation while remaining relatively stable to other reagents. A preferred hydrogenation labile amine protecting group is benzyloxycarbonyl (CBZ).
"Hydrogenation labile acid protecting group" means an acid protecting group as defined above which is readily removed by hydrogenation while remaining relatively stable to other reagents. A preferred hydrogenation labile acid protecting group is benzyl.
"Analogue" means a compound which comprises a chemically modified form of a specific compound or class thereof, and which maintains the pharmaceutical and/or pharmacological activities characteristic of said compound or class.
"Aralkenyl" means an aryl-alkenyl- group wherein the aryl and alkenyl are as herein described. Preferred aralkenyls contain a lower alkenyl moiety. An exemplary aralkenyl group is 2-phenethenyl.
"Aralkoxy" means an aralkyl-O- group wherein the aralkyl groups is as herein described. Exemplary aralkoxy groups include benzyloxy and 1- or 2-naphthalenemethoxy.
"Aralkoxyalkyl" means an aralkyl-O-alkyl group wherein the aralkyl and alkyl groups are as herein described. An exemplary aralkyloxyalkyl group is benzyloxyethyl.
"Aralkoxycarbonyl" means an aralkyl-O-CO- group wherein the aralkyl groups is as herein described. An exemplary aralkoxycarbonyl group is benzyloxycarbonyl.
"Aralkoxycarbonylalkyl" means an aralkyl-OOC-alky- group wherein the alkyl and aralkyl groups are as herein described. Preferred groups include benzyloxy- methyl and ethyl.
"Aralkyl" means an alkyl group substituted by one or more aryl groups, wherein the aryl and alkyl are as herein described. Preferred aralkyls contain a lower alkyl moiety. Exemplary aralkyl groups include benzyl, 2,2-diphenylethyl, 2,2-diphenylmethyl, 2-phenethyl and naphthlenemethyl.
"Aralkylamino" means an aryl-alkyl-NH- group wherein aryl and alkyl are as defined herein.
"Aralkyloxyalkenyl" means an aralkyl-O-alkenyl group wherein the aralkyl and alkenyl groups are as herein described. An exemplary aralkyloxyalkenyl group is 3-benzyloxyallyl.
"Aralkylsulfonyl" means an aralkyl-SO₂- group wherein the aralkyl group is as herein described.
"Aralkylsulfinyl" means an aralkyl-SO- group wherein the aralkyl group is as herein described.
"Aralkylthio" means an aralkyl-S- group wherein the aralkyl group is as herein described. An exemplary aralkylthio group is benzylthio.
"Aroyl" means an aryl-CO- group wherein the aryl group is as herein described. Exemplary groups include benzoyl and 1- and 2-naphthoyl.
"Aroylamino" is an aroyl-NH- group wherein aroyl is as defined herein.
"Aryl" means an aromatic monocyclic or multicyclic ring system of about 6 to about 14 carbon atoms, preferably of about 6 to about 10 carbon atoms. The aryl is optionally substituted with one or more "ring system substituents" which may be the same or different, and are as defined herein. Representative aryl groups include phenyl or naphthyl, or phenyl substituted or naphthyl substituted groups. Preferred aryl groups are phenyl or naphthyl.
"Aralkenyl" means an aryl-alkenyl- group wherein the aryl and alkenyl moiety are as described herein. Preferred alkenyl groups contain a C₂₋₁₂ alkenyl moiety. Exemplary arylalkenyl groups include styryl, 4-phenyl-1,3-pentadienyl, 2,5-dimethyl-2-phenyl-4-hexenyl,
"Aralkynyl" means an aryl-alkynyl- group wherein the aryl and alkynyl moiety are as described herein. Exemplary arylalkynyl groups include phenylacetylene and 3-phenylbut-2-ynyl.
"Aryldiazo" means an aryl-azo- group wherein the aryl and azo groups are as defined herein.
"Arylcarbamoyl" is an aryl-NHCO- group, wherein the aryl group is as defined herein.
"Fused arylcycloalkenyl" means a fused aryl and cycloalkenyl as defined herein. Preferred fused arylcycloalkenyls are those wherein the aryl thereof is phenyl and the cycloalkenyl consists of about 5 to about 6 ring atoms. A fused arylcycloalkenyl as a variable may be bonded through any atom of the ring system thereof capable of such. The fused arylcycloalkenyl may be optionally substituted by one or more ring system substituent, wherein the "ring system substituent" is as defined herein. Representative fused arylcycloalkenyl include 1,2-dihydronaphthylene, indene, and the like.
"Fused arylcycloalkyl" means a fused aryl and cycloalkyl as defined herein. Preferred fused arylcycloalkyls are those wherein the aryl thereof is phenyl and the cycloalkyl consists of about 5 to about 6 ring atoms. A fused arylcycloalkyl as a variable may be bonded through any atom of the ring system thereof capable of such. The fused arylcycloalkyl may be optionally substituted by one or more ring system substituent, wherein the "ring system substituent" is as defined herein. Representative fused arylcycloalkyl includes 1,2,3,4-tetrahydronaphthyl, 5,6,7,8-tetrahydronaphth-1-yl, and the like. Preferred fused arylcycloalkyl include indanyl,
"Fused arylheterocyclenyl" means a fused aryl and heterocyclenyl as defined herein. Preferred fused arylheterocyclenyls are those wherein the aryl thereof is phenyl and the heterocyclenyl consists of about 5 to about 6 ring atoms. A fused arylheterocyclenyl as a variable may be bonded through any atom of the ring system thereof capable of such. The designation of the aza, oxa or thia as a prefix before heterocyclenyl portion of the fused arylheterocyclenyl define that at least a nitrogen, oxygen or sulfur atom is present respectively as a ring atom. The fused arylheterocyclenyl may be optionally substituted by one or more ring system substituent, wherein the "ring system substituent" is as defined herein. The nitrogen atom of a fused arylheterocyclenyl may be a basic nitrogen atom. The nitrogen or sulphur atom of the heterocyclenyl portion of the fused arylheterocyclenyl may also be optionally oxidized to the corresponding N-oxide, S-oxide or S,S-dioxide. Representative fused arylheterocyclenyl include 3H-indolinyl, 1H-2-oxoquinolyl, 2H-1-oxoisoquinolyl, 1,2-dihydroquinolinyl, 3,4-dihydroquinolinyl, indazolyl, 1,2-dihydroisoquinolinyl, benzotriazolyl, ,3,4-dihydroisoquinolinyl, and the like.
"Fused arylheterocyclyl" means a fused aryl and heterocyclyl as defined herein. Preferred fused arylheterocyclyls are those wherein the aryl thereof is phenyl and the heterocyclyl consists of about 5 to about 6 ring atoms. A fused arylheterocyclyl as a variable may be bonded through any atom of the ring system thereof capable of such. The designation of the aza, oxa or thia as a prefix before heterocyclyl portion of the fused arylheterocyclyl define that at least a nitrogen, oxygen or sulfur atom is present respectively as a ring atom. The fused arylheterocyclyl may be optionally substituted by one or more ring system substituent, wherein the "ring system substituent" is as defined herein. The nitrogen atom of a fused arylheteroaryl may be a basic nitrogen atom. The nitrogen or sulphur atom of the heterocyclyl portion of the fused arylheterocyclyl may also be optionally oxidized to the corresponding N-oxide, S-oxide or S,S-dioxide. Representative preferred fused aryllheterocyl ring systems include indolinyl, phthalimide, ,1,2,3,4-tetrahydroisoquinoline, 1,2,3,4-tetrahydroquinoline, 1H-2,3-dihydroisoindol-2-yl, 2,3-dihydrobenz[f]isoindol-2-yl, 1,2,3,4-tetrahydrobenz[g]isoquinolin-2-yl, 1,3-benzodioxole, and the like.
"Aryloxy" means an aryl-O- group wherein the aryl group is as defined herein. Exemplary groups include phenoxy and 2-naphthyloxy.
"Aryloxyalkyl" means an aryl-O-alkyl- group wherein the aryl or alkyl groups are as herein described. An exemplary aryloxyalkyl groups is phenoxypropyl.
"Aryloxyalkenyl" means an aryl-0-alkenyl- group wherein the aryl or alkenyl groups are as herein described. An exemplary aryloxyalkenyl groups is phenoxyallyl.
"Aryloxycarbonyl" means an aryl-O-CO- group wherein the aryl group is as defined herein. Exemplary aryloxycarbonyl groups include phenoxycarbonyl and naphthoxycarbonyl.
"Aryloxycarbonylalkyl" means an aryl-O-OC-alky- group. Preferred groups include phenoxycarbonyl- methyl and ethyl.
"Arylsulfonyl" means an aryl-SO₂- group wherein the aryl group is as defined herein.
"Arylsulfinyl" means an aryl-SO- group wherein the aryl group is as defined herein.
"Arylthio" means an aryl-S- group wherein the aryl group is as herein described. Exemplary arylthio groups include phenylthio and naphthylthio.
"Basic nitrogen atom" means an sp² or sp³ hybridized nitrogen atom having a non-bonded pair of electrons which is capable of being protonated. Examples of basic nitrogen atoms include optionally substituted imino, optionally substituted amino and optionally substituted amidino groups.
"Carbamoyl" is an NH₂-CO- group.
"Carboxy" means a HO(O)C- (carboxylic acid) group.
"Carboxyalkyl" means an HOOC-alkyl- group wherein the alkyl group is as defined herein. Preferred groups include carboxymethyl and carboxyethyl.
"Compounds of the invention", and equivalent expressions, are meant to embrace compounds of general formula (I), and compounds of formula (II), as hereinbefore described, which expression includes the prodrugs, the pharmaceutically acceptable salts, and the solvates, e.g. hydrates, where the context so permits. Similarly, reference to intermediates, whether or not they themselves are claimed, is meant to embrace their salts, and solvates, where the context so permits. For the sake of clarity, particular instances when the context so permits are sometimes indicated in the text, but these instances are purely illustrative and it is not intended to exclude other instances when the context so permits.
"Cycloalkoxy" means an cycloalkyl-O- group wherein the cycloalkyl group is as herein described. Exemplary cycloalkoxy groups include cyclopentyloxy and cyclohexyloxy.
"Cycloalkyl" means a non-aromatic mono- or multicyclic ring system of about 3 to about 10 carbon atoms. Preferred ring sizes of rings of the ring system include about 5 to about 6 ring atoms. The cycloallcyl is optionally substituted with one or more "ring system substituents" which may be the same or different, and are as defined herein. Representative monocyclic cycloalkyl include cyclopropyl, cyclopentyl, cyclohexyl, cycloheptyl, and the like. Representative multicyclic cycloalkyl include 1-decalin, norbornyl, adamant-(1- or 2-)yl, 6,6-dimethylbicyclo[3.1.1]heptane, and the like. Preferred ring system substituents for a cycloalkyl are alkyl, aralkoxy, amidino, hydroxy, or Y¹Y²N- as defined herein.
"Cycloallcylcarbonyl" means a cycloalkyl-CO- group, wherein cycloalkyl is as hereinbefore defined. Exemplary cycloalkylcarbonyl groups include cyclopropylcarbonyl.
"Cycloalkenyl" means a non-aromatic mono- or multicyclic ring system of about 3 to about 10 carbon atoms, preferably of about 5 to about 10 carbon atoms, and which contains at least one carbon-carbon double bond. Preferred ring sizes of rings of the ring system include about 5 to about 6 ring atoms. The cycloalkenyl is optionally substituted with one or more "ring system substituents" which may be the same or different, and are as defined herein. Representative monocyclic cycloalkenyl include cyclopentenyl, cyclohexenyl, cycloheptenyl, and the like. A representative multicyclic cycloalkenyl is norbomylenyl. Preferred ring system substituents for a cycloalkyl are amidino or Y¹Y²N- as defined herein
"Derivative" means a chemically modified compound wherein the modification is considered routine by the ordinary skilled chemist, such as an ester or an amide of an acid, protecting groups, such as a benzyl group for an alcohol or thiol, and tert-butoxycarbonyl group for an amine.
"Diazo" means a bivalent -N=N- radical.
"Effective amount" is means an amount of a compound/composition according to the present invention effective in producing the desired therapeutic effect.
"Electron donating group" shall designate a group that will release or donate electrons more than hydrogen would if it occupied the same position in the molecule. See J. March, Advanced Organic Chemistry, 3rd Ed., John Wiley & Sons p. 238 (1985). These types of groups are well known in the art. Examples include alkyl, aralkyl, cycloalkyl, heteroaralkyl, heteroaryl, or heterocyclyl.
"Formulations suitable for nasal or inhalational administration" means formulations which are in a form suitable to be administered nasally or by inhalation to a patient. The formulation may contain a carrier, in a powder form, having a particle size for example in the range 1 to 500 microns (including particle sizes in a range between 20 and 500 microns in increments of 5 microns such as 30 microns, 35 microns, etc.) Suitable formulations wherein the carrier is a liquid, for administration as for example a nasal spray or as nasal drops, include aqueous or oily solutions of the active ingredient. Formulations suitable for aerosol administration may be prepared according to conventional methods and may be delivered with other therapeutic agents. Inhalational therapy is readily administered by metered dose inhalers.
"Formulations suitable for oral administration" means formulations which are in a form suitable to be administered orally to a patient. The formulations may be presented as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste.
"Formulations suitable for parenteral administration" means formulations which are in a form suitable to be administered parenterally to a patient. The formulations are sterile and include emulsions, suspensions, aqueous and non-aqueous injection solutions, which may contain suspending agents and thickening agents and anti-oxidants, buffers, bacteriostats and solutes which render the formulation isotonic, and have a suitably adjusted pH, with the blood of the intended recipient.
"Formulations suitable for rectal administrations" means formulations which are in a form suitable to be administered rectally to a patient. The formulation is preferably in the form of suppositories which can be prepared by mixing the compounds of this invention with suitable non-irritating excipients or carriers such as cocoa butter, polyethylene glycol or a suppository wax, which are solid at ordinary temperatures but liquid at body temperature and therefore, melt in the rectum or vaginal cavity and release the active component.
"Formulations suitable for systemic administration" means formulations which are in a form suitable to be administered systemically to a patient. The formulation is preferably administered by injection, including transmuscular, intravenous, intraperitoneal, and subcutaneous. For injection, the compounds of the invention are formulated in liquid solutions, preferably in physiologically compatible buffers such as Hank's solution or Ringer's solution. In addition, the compounds may be formulated in solid form and redissolved or suspended immediately prior to use. Lyophilized forms are also included. Systematic administration also can be by transmucosal or transdermal means, or the compounds can be administered orally. For transmucosal or transdermal administration, penetrants appropriate to the barrier to be permeated are used in the formulation. Such penetrants are generally known in the art, and include, for example, bile salts and fusidic acid derivatives for transmucosal administration. In addition, detergents may be used to facilitate permeation. Transmucosal administration may be through use of nasal sprays, for example, or suppositories. For oral administration, the compounds are formulated into conventional oral administration forms such as capsules, tablets, and tonics.
"Formulations suitable for topical administration" means formulations which are in a form suitable to be administered topically to a patient. The formulation may be presented as a topical ointment, salves, powders, sprays and inhalants, gels (water or alcohol based), creams, as is generally known in the art, or incorporated into a matrix base for application in a patch, which would allow a controlled release of compound through the transdermal barrier. When formulated in an ointment, the active ingredients may be employed with either a paraffinic or a water-miscible ointment base. Alternatively, the active ingredients may be formulated in a cream with an oil-in-water cream base. Formulations suitable for topical administration in the eye include eye drops wherein the active ingredient is dissolved or suspended in a suitable carrier, especially an aqueous solvent for the active ingredient. Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.
"Formulations suitable for vaginal administration" means formulations which are in a form suitable to be administered vaginally to a patient. The formulation may be presented as pessaries, tampons, creams, gels, pastes, foams or spray formulations containing in addition to the active ingredient such carriers as are known in the art to be appropriate.
"Halo" means fluoro, chloro, bromo, or iodo. Preferred are fluoro, chloro or bromo, and more preferred are fluoro or chloro.
"Heteroaralkenyl" means an heteroaryl-alkenyl- group wherein the heteroaryl and alkenyl are as herein described. Preferred heteroaralkenyls contain a lower alkenyl moiety. An exemplary aralkenyl group is 4-pyridylvinyl, thienylethenyl, pyridylethenyl, imidazolylethenyl and pyrazinylethenyl.
"Heteroaralkyl" means a heteroaryl-alkyl- group wherein the heteroaryl and alkyl are as herein described. Preferred heteroaralkyls contain a lower alkyl moiety. Exemplary heteroaralkyl groups may contain thienylmethyl, pyridylmethyl, imidazolylmethyl and pyrazinylmethyl.
"Heteroaralkyloxy" means an heteroaralkyl-O- group wherein the heteroaralkyl group is as herein described. An exemplary heteroaralkyloxy group is 4-pyridylmethyloxy.
"Heteroaralkyloxyalkenyl" means an heteroaralkyl-O-alkenyl group wherein the heteroaralkyl and alkenyl groups are as herein described. An exemplary heteroaralkyloxyalkenyl group is 4-pyridylmethyloxyallyl.
"Heteroaralkyloxyalkyl" means an heteroaralkyl-O-alkyl group wherein the heteroaralkyl and alkyl groups are as herein described. An exemplary heteroaralkyloxy group is 4-pyridylmethyloxyethyl.
"Heteroaralkynyl" means an heteroaryl-alkynyl- group wherein the heteroaryl and alkynyl are as herein described. Preferred heteroaralkynyls contain a lower alkynyl moiety. Exemplary heteroaralkynyl groups are pyrid-3-ylacetylenyl and quinolin-3-ylacetylenyl and 4-pyridylethynyl.
"Heteroaroyl" means an means an heteroaryl-CO- group wherein the heteroaryl group is as herein described. Exemplary groups include thiophenoyl, nicotinoyl, pyrrol-2-ylcarbonyl and 1- and 2-naphthoyl and pyridinoyl.
"Heteroaryl" means an aromatic monocyclic or multicyclic ring system of about 5 to about 14 carbon atoms, preferably about 5 to about 10 carbon atoms, in which one or more of the carbon atoms in the ring system is/are hetero element(s) other than carbon, for example nitrogen, oxygen or sulfur. Preferred ring sizes of rings of the ring system include about 5 to about 6 ring atoms. The "heteroaryl" may also be substituted by one or more "ring system substituents" which may be the same or different, and are as defined herein. The designation of the aza, oxa or thia as a prefix before heteroaryl define that at least a nitrogen, oxygen or sulfur atom is present respectively as a ring atom. A nitrogen atom of an heteroaryl may be a basic nitrogen atom and may also be optionally oxidized to the corresponding N-oxide. Representative heteroaryl and substituted heteroaryl groups include pyrazinyl, furanyl, thienyl, pyridyl, pyrimidinyl, isoxazolyl, isothiazolyl, tetrazolyl, oxazolyl, thiazolyl, pyrazolyl, furazanyl, pyrrolyl, pyrazolyl, triazolyl, 1,2,4-thiadiazolyl, pyridazinyl, quinoxalinyl, phthalazinyl, imidazo[1,2-a]pyridine, imidazo[2,1-b]thiazolyl, benzofurazanyl, indolyl, azaindolyl, benzimidazolyl, benzothienyl, quinolinyl, imidazolyl, thienopyridyl, quinazolinyl, thienopyrimidyl, pyrrolopyridyl, imidazopyridyl, isoquinolinyl, benzoazaindole, 1,2,4-triazinyl, Preferred heteroaryl groups include pyrazinyl, thienyl, pyridyl, pyrimidinyl, quinolinyl, tetrazolyl, imidazolyl, thiazolyl, benzothienyl, isoxazolyl and isothiazolyl.
"Heteroarylalkenyl" means a heteroaryl-alkenyl-group wherein the heteroaryl and alkenyl moieties are as described herein. Preferred heteroarylalkenyl groups contain a C₂₋₁₂ alkenyl moiety. Exemplary heteroarylalkenyl groups include pyridylpentenyl, pyridylhexenyl and pyridylheptenyl.
"Heteroarylalkynyl" means an aryl-alkynyl- group wherein the heteroaryl and alkynyl moiety are as herein described. Preferred heteroarylalkynyl groups contain a C₂₋₁₂ alkynyl moiety. Exemplary heteroarylalkynyl groups include 3-pyridyl-but-2-ynyl and pyridylpropynyl.
"Heteroaryldiazo" means an heteroaryl -azo- group wherein the heteroaryl and azo groups are as defined herein.
"Fused heteroarylcycloalkenyl" means a fused heteroaryl and cycloalkenyl as defined herein. Preferred fused heteroarylcycloalkenyls are those wherein the heteroaryl thereof is phenyl and the cycloakenyl consists of about 5 to about 6 ring atoms. A fused heteroarylcycloalkenyl as a variable may be bonded through any atom of the ring system thereof capable of such. The designation of the aza, oxa or thia as a prefix before heteroaryl portion of the fused heteroarylcycloalkenyl define that at least a nitrogen, oxygen or sulfur atom is present respectively as a ring atom. The fused heteroarylcycloalkenyl may be optionally substituted by one or more ring system substituent, wherein the "ring system substituent" is as defined herein. The nitrogen atom of a fused heteroarylcycloalkenyl may be a basic nitrogen atom. The nitrogen atom of the heteroaryl portion of the fused heteroarylcycloalkenyl may also be optionally oxidized to the corresponding N-oxide. Representative fused heteroarylcycloalkenyl include 5,6-dihydroquinolyl, 5,6-dihydroisoquinolyl, 5,6-dihydroquinoxalinyl, 5,6-dihydroquinazolinyl, 4,5-dihydro-1H-benzimidazolyl, 4,5-dihydrobenzoxazolyl, and the like.
"Fused heteroarylcycloalkyl" means a fused heteroaryl and cycloalkyl as defined herein. Preferred fused heteroarylcycloalkyls are those wherein the heteroaryl thereof consists of about 5 to about 6 ring atoms and the cycloalkyl consists of about 5 to about 6 ring atoms. A fused heteroarylcycloalkyl as a variable may be bonded through any atom of the ring system thereof capable of such. The designation of the aza, oxa or thia as a prefix before heteroaryl portion of the fused heteroarylcycloalkyl define that at least a nitrogen, oxygen or sulfur atom is present respectively as a ring atom. The fused heteroarylcycloalkyl may be optionally substituted by one or more ring system substituent, wherein the "ring system substituent" is as defined herein. The nitrogen atom of a fused heteroarylcycloalkyl may be a basic nitrogen atom. The nitrogen atom of the heteroaryl portion of the fused heteroarylcycloalkyl may also be optionally oxidized to the corresponding N-oxide. Representative fused heteroarylcycloalkyl include 5,6,7,8-tetrahydroquinolinyl, 5,6,7,8-tetrahydroisoquinolyl, 5,6,7,8-tetrahydroquinoxalinyl, 5,6,7,8-tetrahydroquinazolyl, 4,5,6,7-tetrahydro-1H-benzimidazolyl, 4,5,6,7-tetrahydrobenzoxazolyl, 1H-4-oxa-1,5-diazanaphthalen-2-onyl, 1,3-dihydroimidizole-[4,5]-pyridin-2-onyl, and the like.
"Fused heteroarylheterocyclenyl" means a fused heteroaryl and heterocyclenyl as defined herein. Preferred fused heteroarylheterocyclenyls are those wherein the heteroaryl thereof consists of about 5 to about 6 ring atoms and the heterocyclenyl consists of about 5 to about 6 ring atoms. A fused heteroarylheterocyclenyl as a variable may be bonded through any atom of the ring system thereof capable of such. The designation of the aza, oxa or thia as a prefix before the heteroaryl or heterocyclenyl portion of the fused heteroarylheterocyclenyl define that at least a nitrogen, oxygen or sulfur atom is present respectively as a ring atom. The fused heteroarylheterocyclenyl may be optionally substituted by one or more ring system substituent, wherein the "ring system substituent" is as defined herein. The nitrogen atom of a fused heteroarylazaheterocyclenyl may be a basic nitrogen atom. The nitrogen or sulphur atom of the heteroaryl portion of the fused heteroarylheterocyclyl may also be optionally oxidized to the corresponding N-oxide. The nitrogen or sulphur atom of the heteroaryl or heterocyclyl portion of the fused heteroarylheterocyclyl may also be optionally oxidized to the corresponding N-oxide, S-oxide or S,S-dioxide. Representative fused heteroarylheterocyclenyl include 7,8-dihydro[1,7]naphthyridinyl, 1,2-dihydro[2,7]naphthyridinyl, 6,7-dihydro-3H-imidazo[4,5-c]pyridyl, 1,2-dihydro-1,5-naphthyridinyl, 1,2-dihydro-1,6-naphthyridinyl, 1,2-dihydro-1,7-naphthyridinyl, 1,2-dihydro-1,8-naphthyridinyl, 1,2-dihydro-2,6-naphthyridinyl, and the like.
"Fused heteroarylheterocyclyl" means a fused heteroaryl and heterocyclyl as defined herein. Preferred fused heteroarylheterocyclyls are those wherein the heteroaryl thereof consists of about 5 to about 6 ring atoms and the heterocyclyl consists of about 5 to about 6 ring atoms. A fused heteroarylheterocyclyl as a variable may be bonded through any atom of the ring system thereof capable of such. The designation of the aza, oxa or thia as a prefix before the heteroaryl or heterocyclyl portion of the fused heteroarylheterocyclyl define that at least a nitrogen, oxygen or sulfur atom is present respectively as a ring atom. The fused heteroarylheterocyclyl may be optionally substituted by one or more ring system substituent, wherein the "ring system substituent" is as defined herein. The nitrogen atom of a fused heteroarylheterocyclyl may be a basic nitrogen atom. The nitrogen or sulphur atom of the heteroaryl portion of the fused heteroarylheterocyclyl may also be optionally oxidized to the corresponding N-oxide. The nitrogen or sulphur atom of the heteroaryl or heterocyclyl portion of the fused heteroarylheterocyclyl may also be optionally oxidized to the corresponding N-oxide, S-oxide or S,S-dioxide. Representative fused heteroarylheterocyclyl include 2,3-dihydro-1H pyrrol[3,4-b]quinolin-2-yl, 1,2,3,4-tetrahydrobenz [b][1,7]naphthyridin-2-yl, 1,2,3,4-tetrahydrobenz [b][1,6]naphthyridin-2-yl, 1,2,3,4-tetrahydro-9H-pyrido[3,4-b]indol-2yl, 1,2,3,4-tetrahydro-9H-pyrido[4,3-b]indol-2y1, 2,3,-dihydro-1H-pyrrolo[3,4-b]indol-2-yl, 1H-2,3,4,5-tetrahydroazepino[3,4-b]indol-2-yl, 1H-2,3,4,5-tetrahydroazepino[4,3-b]indol-3-yl, 1H-2,3,4,5-tetrahydroazepino[4,5-b]indol-2 yl, 5,6,7,8-tetrahydro[1,7]napthyridinyl, 1,2,3,4-tetrhydro[2,7]naphthyridyl, 2,3-dihydro[1,4]dioxino[2,3-b]pyridyl, 2,3-dihydro[1,4]dioxino[2,3-b]pryidyl, 3,4-dihydro-2H-1-oxa[4,6]diazanaphthalenyl, 4,5,6,7-tetrahydro-3H-imidazo[4,5-c]pyridyl, 6,7-dihydro[5,8]diazanaphthalenyl, 1,2,3,4-tetrahydro[1,5] napthyridinyl, 1,2,3,4-tetrahydro[1,6]napthyridinyl, 1,2,3,4-tetrahydro[1,7]napthyridinyl, 1,2,3,4-tetrahydro[1,8]napthyridinyl, 1,2,3,4-tetrahydro[2,6]napthyridinyl, and the like.
"Heteroarylsulphonylcarbamoyl" means a heteroaryl-SO₂-NH-C(=O)- group wherein the heteroaryl group is as herein described.
"Heterocyclenyl" means a non-aromatic monocyclic or multicyclic hydrocarbon ring system of about 3 to about 13 carbon atoms, preferably about 5 to about 13 carbon atoms, in which one or more of the carbon atoms in the ring system is/are hetero element(s) other than carbon, for example nitrogen, oxygen or sulfur atoms, and which contains at least one carbon-carbon double bond or carbon-nitrogen double bond. Preferred ring sizes of rings of the ring system include about 5 to about 6 ring atoms. The designation of the aza, oxa or thia as a prefix before heterocyclenyl define that at least a nitrogen, oxygen or sulfur atom is present respectively as a ring atom. The heterocyclenyl may be optionally substituted by one or more ring system substituent, wherein the "ring system substituent" is as defined herein. The nitrogen atom of an heterocyclenyl may be a basic nitrogen atom. The nitrogen or sulphur atom of the heterocyclenyl may also be optionally oxidized to the corresponding N-oxide, S-oxide or S,S-dioxide. Representative monocyclic azaheterocyclenyl groups include 1,2,3,4- tetrahydrohydropyridine, 1,2-dihydropyridyl, 1,4-dihydropyridyl, 1,2,3,6-tetrahydropyridine, 1,4,5,6-tetrahydropyrimidine, 2-pyrrolinyl, 3-pyrrolinyl, 2-imidazolinyl, 2-pyrazolinyl, 1,4,4a,5a,6,9,9a,9b-octahydro-dibenzofuran, and the like. Exemplary oxaheterocyclenyl groups include 3,4-dihydro-2*H*-pyran, dihydrofuranyl, and fluorodihydrofuranyl. Preferred is dihydrofuranyl. An exemplary multicyclic oxaheterocyclenyl group is 7-oxabicyclo[2.2.1]heptenyl. Preferred monocyclic thiaheterocycleny rings include dihydrothiophenyl and dihydrothiopyranyl; more preferred is dihydrothiophenyl. Preferred ring system substituents include amidino, halogen, hydroxy, alkoxycarbonylalkyl, carboxyalkyl or Y¹Y²N- as defined herein.
"Heterocyclyl" means a non-aromatic saturated monocyclic or multicyclic ring system of about 3 to about 10 carbon atoms, preferably about 5 to about 10 carbon atoms, in which one or more of the carbon atoms in the ring system is/are hetero element(s) other than carbon, for example nitrogen, oxygen or sulfur. Preferred ring sizes of rings of the ring system include about 5 to about 6 ring atoms. The designation of the aza, oxa or thia as a prefix before heterocyclyl define that at least a nitrogen, oxygen or sulfur atom is present respectively as a ring atom. The heterocyclyl may be optionally substituted by one or more "ring system substituents" which may be the same or different, and are as defined herein. The nitrogen atom of an heterocyclyl may be a basic nitrogen atom. The nitrogen or sulphur atom of the heterocyclyl may also be optionally oxidized to the corresponding N-oxide, S-oxide or S,S-dioxide. Representative monocyclic heterocyclyl rings include piperidyl, pyrrolidinyl, piperazinyl, morpholinyl, thiomorpholinyl, thiazolidinyl, 1,3-dioxolanyl, 1,4-dioxanyl, tetrahydrofuranyl, tetrahydrothiophenyl, 2-thioxo-4-thiazolidinonyl, tetrahydrothiopyranyl, and the like. Preferred heterocyclyl groups include pyrrolidinyl, tetrahydrofuranyl, morpholinyl, piperidyl, Preferred heterocyclyl group substituents include alkyl, aralkyl, amidino, halogen, hydroxy, aralkoxycarbonyl, alkoxycarbonylalkyl, carboxyalkyl or Y¹Y²N-as defined herein.
"Heterocyclylalkyl" means an heterocyclyl-alkyl- group wherein the heterocyclyl and alkyl are as herein described. Preferred heterocyclylalkyls contain a lower alkyl moiety. An exemplary heteroaralkyl group is tetrahydropyranylmethyl.
"Heterocyclylalkyloxyalkyl" means an heterocyclyl-alkyl-0-alkyl- group wherein the heterocyclyl and alkyls groups independently are as herein described. An exemplary heteroaralkyl group is tetrahydropyranylmethyloxymethyl.
"Heterocyclyloxy" means a heterocyclyl-O- group in which the heterocyclyl group is as previously described. Exemplary heterocyclyloxy groups include quinuclidyloxy, pentamethylenesulfideoxy, tetrahydropyranyloxy, tetrahydrothiophenyloxy, pyrrolidinyloxy, tetrahydrofuranyloxy or 7-oxabicyclo[2.2.1]heptanyloxy, hydroxytetrahydropyranyloxy and hydroxy-7-oxabicyclo[2.2.1]heptanyloxy.
"Hydrate" means a solvate wherein the solvent molecule(s) is/are H₂O.
"Hydroxyalkyl" means a HO-alkyl- group wherein alkyl is as herein defined. Preferred hydroxyalkyls contain lower alkyl. Exemplary hydroxyalkyl groups include hydroxymethyl and 2-hydroxyethyl.
"Hygroscopicity" means sorption, implying an acquired amount or state of water sufficient to affect the physical or chemical properties of the substance (Eds. J. Swarbrick and J. C. Boylan, Encyclopedia of Pharmaceutical Technology, Vol. 10, p. 33).
"Liquid dosage form" means the dose of the active compound to be administered to the patient is in liquid form, for example, pharmaceutically acceptable emulsions, solutions, suspensions, syrups and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil and sesame oil, glycerol, tetrahydrofurfuryl alcohol, polyethylene glycols and fatty acid esters of sorbitan or mixtures of these substances, and the like.
"Modulate" refers to the ability of a compound to either directly (by binding to the receptor as a ligand) or indirectly (as a precursor for a ligand or an inducer which promotes production of a ligand from a precursor) induce expression of gene(s) maintained under hormone control, or to repress expression of gene (s) maintained under such control.
"Patient" includes both human and other mammals.
"Pharmaceutical composition" refers to a composition comprising a compound of formula (I) and at least one component selected from the group comprising pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms. Examples of suspending agents include ethoxylated isostearyl alcohols, polyoxyethylene sorbitol and sorbitan esters, microcrystalline cellulose, aluminum metahydroxide, bentonite, agar-agar and tragacanth, or mixtures of these substances. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monosterate and gelatin. Examples of suitable carriers, diluents, solvents or vehicles include water, ethanol, polyols, suitable mixtures thereof, vegetable oils (such as olive oil) and injectable organic esters such as ethyl oleate. Examples of excipients include lactose, milk sugar, sodium citrate, calcium carbonate, dicalcium phosphate phosphate. Examples of disintegrating agents include starch, alginic acids and certain complex silicates. Examples of lubricants include magnesium stearate, sodium lauryl sulphate, talc, as well as high molecular weight polyethylene glycols.
"Pharmaceutically acceptable" means it is, within the scope of sound medical judgment, suitable for use in contact with the cells of humans and lower animals without undue toxicity, irritation, allergic response and the like, and are commensurate with a reasonable benefit/risk ratio.
"Pharmaceutically acceptable dosage forms" refers to dosage forms of the compound of the invention, and includes, for example, tablets, dragees, powders, elixirs, syrups, liquid preparations, including suspensions, sprays, inhalants tablets, lozenges, emulsions, solutions, granules, capsules and suppositories, as well as liquid preparations for injections, including liposome preparations. Techniques and formulations generally may be found in Remington's Pharmaceutical Sciences, Mack Publishing Co., Easton, PA, latest edition.
"Pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyates, acrylates and ethylsuccinates.
"Pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the above formula, for example by hydrolysis in blood. Functional groups which may be rapidly transformed, by metabolic cleavage, in vivo form a class of groups reactive with the carboxyl group of the compounds of this invention. They include, but are not limited to such groups as alkanoyl (such as acetyl, propionyl, butyryl, and the like), unsubstituted and substituted aroyl (such as benzoyl and substituted benzoyl), alkoxycarbonyl (such as ethoxycarbonyl), trialkylsilyl (such as trimethyl- and triethysilyl), monoesters formed with dicarboxylic acids (such as succinyl), and the like. Because of the ease with which the metabolically cleavable groups of the compounds of this invention are cleaved in vivo, the compounds bearing such groups act as pro-drugs. The compounds bearing the metabolically cleavable groups have the advantage that they may exhibit improved bioavailability as a result of enhanced solubility and/or rate of absorption conferred upon the parent compound by virtue of the presence of the metabolically cleavable group. A thorough discussion is provided in Design of Prodrugs, H. Bundgaard, ed., Elsevier, 1985; Methods in Enzymology, . K. Widder et al, Ed., Academic Press, 42, p.309-396, 1985; A Textbook of Drug Design and Developement, Krogsgaard-Larsen and H. Bundgaard, ed., Chapter 5; "Design and Applications of Prodrugs" p.113-191, 1991; Advanced Drug Delivery Reviews, H. Bundgard, 8, p.1-38, 1992; Journal of Pharmaceutical Sciences, 77, p. 285, 1988; Chem. Pharm. Bull., N. Nakeya et al, 32, p. 692, 1984; Pro-drugs as Novel Delivery Systems, T. Higuchi and V. Stella, Vol. 14 of the A.C.S. Symposium Series, and Bioreversible Carriers in Drug Design, Edward B. Roche, ed., American Pharmaceutical Association and Pergamon Press, 1987, which are incorporated herein by reference.
"Pharmaceutically acceptable salts" refers to the relatively non-toxic, inorganic and organic acid addition salts, and base addition salts, of compounds of the present invention. These salts can be prepared *in situ* during the final isolation and purification of the compounds. In particular, acid addition salts can be prepared by separately reacting the purified compound in its free base form with a suitable organic or inorganic acid and isolating the salt thus formed. Representative acid addition salts include the hydrobromide, hydrochloride, sulfate, bisulfate, phosphate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, sulphamates, malonates, salicylates, propionates, methylene-bis-β-hydroxynaphthoates, gentisates, isethionates, di-p-toluoyltartrates, methane-sulphonates, ethanesulphonates, benzenesulphonates, p-toluenesulphonates, cyclohexylsulphamates and quinateslaurylsulphonate salts, and the like. (See, for example S. M. Berge, et al., "Pharmaceutical Salts," J. Pharm. Sci., 66: p.1-19 (1977) which is incorporated herein by reference.) Base addition salts can also be prepared by separately reacting the purified compound in its acid form with a suitable organic or inorganic base and isolating the salt thus formed. Base addition salts include pharmaceutically acceptable metal and amine salts. Suitable metal salts include the sodium, potassium, calcium, barium, zinc, magnesium, and aluminum salts. The sodium and potassium salts are preferred. Suitable inorganic base addition salts are prepared from metal bases which include sodium hydride, sodium hydroxide, potassium hydroxide, calcium hydroxide, aluminium hydroxide, lithium hydroxide, magnesium hydroxide, zinc hydroxide. Suitable amine base addition salts are prepared from amines which have sufficient basicity to form a stable salt, and preferably include those amines which are frequently used in medicinal chemistry because of their low toxicity and acceptability for medical use. ammonia, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, e.g., lysine and arginine, and dicyclohexylamine, and the like.
"Solid dosage form" means the dosage form of the compound of the invention is solid form, for example capsules, tablets, pills, powders, dragees or granules. In such solid dosage forms, the compound of the invention is admixed with at least one inert customary excipient (or carrier) such as sodium citrate or dicalcium phosphate or (a) fillers or extenders, as for example, starches, lactose, sucrose, glucose, mannitol and silicic acid, (b) binders, as for example, carboxymethylcellulose, alignates, gelatin, polyvinylpyrrolidone, sucrose and acacia, (c) humectants, as for example, glycerol, (d) disintegrating agents, as for example, agar-agar, calcium carbonate, potato or tapioca starch, alginic acid, certain complex silicates and sodium carbonate, (e) solution retarders, as for example paraffin, (f) absorption accelerators, as for example, quaternary ammonium compounds, (g) wetting agents, as for example, cetyl alcohol and glycerol monostearate, (h) adsorbents, as for example, kaolin and bentonite, (i) lubricants, as for example, talc, calcium stearate, magnesium stearate, solid polyethylene glycols, sodium lauryl sulfate, (j) opacifying agents, (k) buffering agents , and agents which release the compound(s) of the invention in a certain part of the intestinal tract in a delayed manner.
"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolable solvates. Representative solvates include ethanolates, methanolates, and the like.
"Ring system substituents" mean substituents attached to aromatic or non-aromatic ring systems inclusive of hydrogen, alkyl, alkenyl, alkynyl, aryl, heteroaryl, aralkyl, heteroaralkyl, hydroxy, hydroxyalkyl, alkoxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, aryldiazo, heteroaryldiazo, amidino, Y¹Y²N-, Y¹Y²N-alkyl-, Y¹Y²NCO- or Y¹Y²NSO₂-, wherein Y¹ and Y² are independently hydrogen, optionally substituted alkyl, optionally substituted aryl, optionally substituted aralkyl or optionally substituted heteroaralkyl, or for where the substituent is Y¹Y²N-, then one of Y¹ and Y² may be acyl or aroyl as defined herein and the other of Y¹ and Y² is as defined previously, or for where the substituent is Y¹Y²NCO- or Y¹Y²NSO₂-, Y¹ and Y² may also be taken together with the N atom through which Y¹ and Y² are linked to form a 4 to 7 membered heterocyclyl or heterocyclenyl. Preferred ring system substituents are alkoxycarbonyl, alkoxy, halo, aryl, aralkoxy, alkyl, hydroxy, aryloxy, nitro, alkylsulfonyl, heteroaryl, Y¹Y²N-. Most preferred ring system substituents are selected from alkoxycarbonyl, halo, aryl, aralkoxy, aralkyl, alkyl, hydroxy, aryloxy, Y¹Y²N-, oxo, cyano, nitro, and arylsulfinyl, . When a ring system is saturated or partially saturated, the "ring system substituents" further include , methylene (H₂C=), oxo (O=), thioxo (S=).
"Y¹Y²N-" means a substituted or unsubstituted amino group, wherein Y¹ and Y² are as herein described. Exemplary groups include amino (H₂N-), methylamino, dimethylamino, diethylamino, pyrrolidine, piperidine, benzylamino, or phenethylamino.
"Y¹Y²NCO-" means a substituted or unsubstituted carbamoyl group, wherein Y¹ and Y² are as herein described. Exemplary groups are carbamoyl (H₂NCO-) and dimethylaminocarbamoyl (Me₂NCO-).
"Y¹Y²NSO₂-" means a substituted or unsubstituted sulfamoyl group, wherein Y¹ and Y² are as herein described. Exemplary groups are aminosulfamoyl (H₂NSO₂-) and dimethylaminosulfamoyl (Me₂NSO₂-).
"Primary or secondary protected amine" means a group of the following formula Y^{a}Y^{b}N- wherein one of Y^{a} and Y^{b} is P^{a} a nitrogen protecting group and the other of Y^{a} and Y^{b} is hydrogen, alkenyl, alkyl, aralkyl, aryl, fused arylcycloalkenyl, fused arylcycloalkyl, fused arylheterocyclenyl, fused arylheterocyclyl, cycloalkyl, cycloalkenyl, heteroaralkyl, heteroaryl, fused heteroarylcycloalkenyl, fused heteroarylcycloalkyl, fused heteroarylheterocyclenyl, fused heteroarylheterocyclyl, heterocyclenyl or heterocyclyl.
"Activated carboxylic acid" means a group of the following formula LO-CO-wherein L is aliphatic, aromatic or a resin moiety.

In a specific embodiment, the term "about" or "approximately" means within 20%, preferably within 10%, and more preferably within 5% of a given value or range.

### Preferred Embodiments

One particular aspect of the present invention is directed to a solid phase synthesis of a compound of formula (I) via a '3-step, one pot' procedure, employing the Ugi multi-component reaction (MCR) (Ugi, I., Angew. Chem. Int. Ed. Engl., 1962, 1, 8) combining reacting a nitrogen-protected amino acid of formula (XIV), with an aldehyde or ketone of formula (XV), an amine of formula (XVI), and a resin bound isonitrile selected from (IXa) or (XVIII) to form the respective intermediate resin bound compound and nitrogen-deprotection of the intermediate compound and cyclization to form the compound of formula (I).

In another aspect, this invention is directed to the preparation of 1,4-benzodiazepine-2,5-dione derivatives of general formulae (I), by solid phase synthesis employing the Ugi multi-component reaction (MCR) (Ugi, I., Angew. Chem. Int. Ed. Engl., 1962, 1, 8) using an isonitrile functionalized polymer resin linker (IXa) as described herein, followed by amine deprotection, cleavage from the resin and cyclization. The alkoxide and hydroxide safety-catch clipping strategy and subsequent solution phase cyclization offers similar advantages to a traceless linker (Plunkett, M.J.; Ellman, J.A. J. Org. Chem. 1995, 60, 6006; Hulme, C.; Peng, J.; Morton, G.; Salvino, J.M.; Herpin, T.; Labaudiniere, R. Tetrahedron Lett. 1998, 39,) in that no constant functionality derived from clipping remains at the end of the synthetic protocol.

In another aspect, this invention is directed to the preparation and use of a novel resin bound isonitrile (IXa), deployed as a novel safety catch linker (Backes, B. J., Virgilio, A. A., Ellman, J. A. J. Am. Chem. Soc. 1996, 118, 3055; Kenner, G. W., McDermott, J. R., Sheppard, R. C. J. Chem. Soc., Chem. Commun. 1971, 636.) in the preparation of 1,4-benzodiazepine-2,5-dione derivatives of general formulae (I).

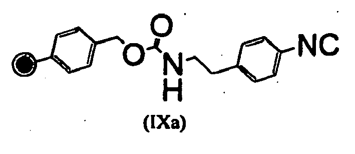

A preferred aspect of the compound are those wherein:
R¹ is aralkyl, alkyl, aryl, heteroaryl, cycloalkyl, aralkenyl, heterocyclenyl or heterocyclyl.

A preferred aspect of the compound are those wherein:
R¹ is hydrogen or alkyl.

A preferred aspect of the compound are those wherein:
R² represents heteroaralkyl, aralkyl, alkyl, fused arylcycloalkyl, cycloalkyl, heterocyclyl, aryl, fused arylheterocyclenyl, or fused arylheterocyclyl.

A preferred aspect of the compound are those wherein:
R³ represents hydrogen, alkyl, aralkyl, aryl, fused arylcycloalkenyl, fused arylcycloalkyl, fused arylheterocyclenyl, fused arylheterocyclyl, cycloalkyl, cycloalkenyl, heteroaralkyl, heteroaryl, fused heteroarylcycloalkenyl, fused heteroarylcycloalkyl, fused heteroarylheterocyclenyl, fused heteroarylheterocyclyl, heterocyclenyl, or heterocyclyl

A preferred aspect of the compound are those wherein:
R⁶, R⁷ R⁸ and R^{8'} independently represents hydrogen, alkenyl, alkyl, aryl, aralkyl, heteroaralkyl, hydroxy, aryloxy, alkoxy, aralkoxy, halo, nitro, cyano, alkylsulfonyl, arylsulfonyl, heteroarylsulfonyl, alkylthio, arylthio, heteroarylthio, heteroaralkylthio, cycloalkyl, heterocyclyl, fused arylcycloalkenyl, fused arylcycloalkyl, fused arylheterocyclenyl, fused arylheterocyclyl, fused heteroarylcycloalkenyl, fused heteroarylcycloalkyl, fused heteroarylheterocyclenyl, fused heteroarylheterocyclyl, heteroarylsulphonylcarbamoyl, heteroaryl, Y¹Y²N-, or Y¹Y²NSO₂-.

A preferred aspect of the compound are those wherein:
two adjacent substituents selected from the substituents R⁶, R⁷, R^{8'} and R⁸ taken together with the aryl carbon atoms through which the two adjacent substituents are linked form a 5 to 7 membered cycloalkyl or a cycloalkenyl, heterocyclyl or heterocyclenyl, or 6 membered aryl or 5 to 6 membered heteroaryl.

A more preferred aspect of the compound are those wherein:
R⁹ is hydrogen.

A more preferred aspect of the compound are those wherein:
R⁹ is alkyl.

A more preferred aspect of the compound are those wherein:
R¹ is aralkyl, alkyl, aryl, heteroaryl, cycloalkyl, or heterocyclyl.

A more preferred aspect of the compound are those wherein:
R² represents aralkyl, alkyl, fused arylheterocyclenyl, or fused arylheterocyclyl.

A more preferred aspect of the compound are those wherein:
R³ represents hydrogen, alkyl, aralkyl, cycloalkyl, cycloalkenyl, heteroaralkyl or heterocyclenyl, heterocyclyl.

A more preferred aspect of the compound are those wherein:
R³ represents hydrogen.

A more preferred aspect of the compound are those wherein:
R⁶, R⁷ R⁸ and R^{8'}, independently represents hydrogen, halo, alkoxy, alkyl, fused arylcycloalkenyl, fused arylcycloalkyl, fused arylheterocyclenyl, fused arylheterocyclyl, fused heteroarylcycloalkenyl, fused heteroarylcycloalkyl, fused heteroarylheterocyclenyl, fused heteroarylheterocyclyl, or heteroaryl.

A more preferred aspect of the compound are those wherein:
R¹² represents alkyl, aralkyl, aryl, cycloalkyl, or heterocyclyl.

A preferred compound is selected from the group of formulae consisting of:

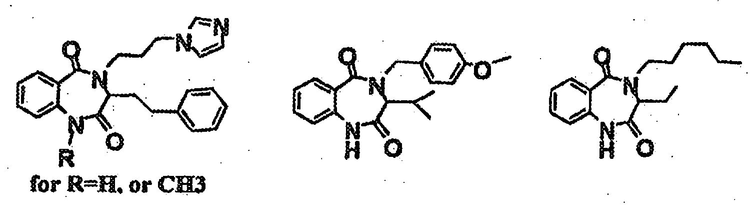

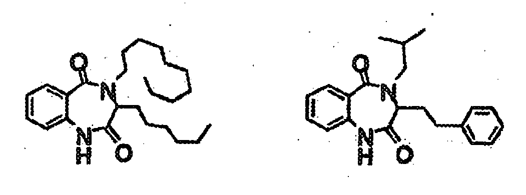

It is to be understood that this invention covers all appropriate combinations of the particular and preferred groupings referred to herein.

Described are also kits having a plurality of active ingredients (with or without carrier) which, together, may be effectively utilized for carrying out novel combination therapies

Described is also a novel pharmaceutical composition which is effective, in and of itself, for utilization in a beneficial combination therapy because it includes a plurality of active ingredients which may be prepared by the invention.

The compounds optionally are supplied as salts. Those salts which are pharmaceutically acceptable are of particular interest since they are useful in administering the foregoing compounds for medical purposes. Salts which are not pharmaceutically acceptable are useful in manufacturing processes, for isolation and purification purposes, and in some instances, for use in separating stereoisomeric forms of the compounds. The latter is particularly true of amine salts prepared from optically active amines.

Where the compound contains a carboxy group, or a sufficiently acidic bioisostere, base addition salts may be formed and are simply a more convenient form for use; and in practice, use of the salt form inherently amounts to use of the free acid form.

Also, where the compound contains a basic group, or a sufficiently basic bioisostere, acid addition salts may be formed and are simply a more convenient form for use; and in practice, use of the salt form inherently amounts to use of the free base form.

The foregoing compounds may also be mixed another therapeutic compound to form pharmaceutical compositions (with or without diluent or carrier) which, when administered, provide simultaneous administration of a combination of active ingredients resulting in the combination therapy of the invention.

While it is possible for the compounds to be administered alone it is preferably to present them as pharmaceutical compositions. The pharmaceutical compositions, both for veterinary an for human use, comprise at lease one compound prepared as above defined, together with one or more acceptable carriers therefor and optionally other therapeutic ingredients.

In certain preferred embodiments, active ingredients necessary in combination therapy may be combined in a single pharmaceutical composition for simultaneous administration.

The choice of vehicle and the content of active substance in the vehicle are generally determined in accordance with the solubility and chemical properties of the active compound, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

The oily phase of the emulsions may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the emulsifying wax, and the way together with the oil and fat make up the emulsifying ointment base which forms the oily dispersed phase of the cream formulations. Emulgents and emulsion stabilizers suitable for use in the formulation of the present invention include Tween^{®} 60, Span^{®} 80, cetostearyl alcohol, benzyl alcohol, myristyl alcohol, glyceryl monostearate and sodium lauryl sulfate.

If desired, the aqueous phase of the cream base may include, for example, a least 30% w/w of a polyhydric alcohol, i.e. an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulphoxide and related analogs.

The choice of suitable oils or fats for the formulation is based on achieving the desired cosmetic properties. Thus the cream should preferably be a non-greasy, non-staining and washable product with suitable consistency to avoid leakage from tubes or other containers. Straight or branched chain, mono- or dibasic alkyl esters such as di-isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Solid compositions of may also be employed as fillers in soft and hard-filled gelatin capsules using such excipients as lactose or milk sugar as well as high molecular weight polyethylene glycols, and the like.

The pharmaceutical compositions can be administered in a suitable formulation to humans and animals by topical or systemic administration, including oral, inhalational, rectal, nasal, buccal, sublingual, vaginal, parenteral (including subcutaneous, intramuscular, intravenous, intradermal, intrathecal and epidural), intracisternal and intraperitoneal. It will be appreciated that the preferred route may vary with for example the condition of the recipient.

The formulations can be prepared in unit dosage form by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

A tablet may be made by compression or moulding, optionally with one or more accessory ingredients. Compressed tables may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compounds moistened with an inert liquid diluent. The tablets may optionally be coated or scored and may be formulated so as to provide slow or controlled release of the active ingredient therein.

Solid compositions for rectal administration include suppositories formulated in accordance with known methods and containing at least one compound.

If desired, and for more effective distribution, the compounds can be microencapsulated in, or attached to, a slow release or targeted delivery systems such as a biocompatible, biodegradable polymer matrices (e.g. poly(d,l-lactide co-glycolide)), liposomes, and microspheres and subcutaneously or intramuscularly injected by a technique called subcutaneous or intramuscular depot to provide continuous slow release of the compound(s) for a period of 2 weeks or longer. The compounds may be sterilized, for example, by filtration through a bacteria retaining filter, or by incorporating sterilizing agents in the form of sterile solid compositions which can be dissolved in sterile water, or some other sterile injectable medium immediately before use.

Actual dosage levels of active ingredient in the compositions may be varied so as to obtain an amount of active ingredient that is effective to obtain a desired therapeutic response for a particular composition and method of administration. The selected dosage level therefore depends upon the desired therapeutic effect, on the route of administration, on the desired duration of treatment and other factors.

Total daily dose of the compounds administered to a host in single or divided doses may be in amounts, for example, of from about 0.001 to about 100 mg/kg body weight daily and preferably 0.01 to 10 mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

The amount of each component administered is determined by the attending clinicians taking into consideration the etiology and severity of the disease, the patient's condition and age, the potency of each component and other factors.

The formulations may be presented in unit-dose or multi-dose containers, for example sealed ampoules and vials with elastomeric stoppers, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

### Preparation of Compounds of the Invention

The starting materials and intermediates of compounds used in the invention may be prepared by the application or adaptation of known methods, for example methods as described in the Reference Example or their obvious chemical equivalents.

Compounds used in the invention may be prepared by the application or adaptation of known methods, by which is meant methods used heretofore or described in the literature, for example those described by R. C. Larock in Comprehensive Organic Transformations, VCH publishers, 1989.

In the reactions described hereinafter it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Green and P.G.M.Wuts in "Protective Groups in Organic Chemistry" John Wiley and Sons, 1991; J. F. W. McOmie in "Protective Groups in Organic Chemistry" Plenum Press, 1973.

### General methodology for the preparation the isonitrile resin linker (IXa)

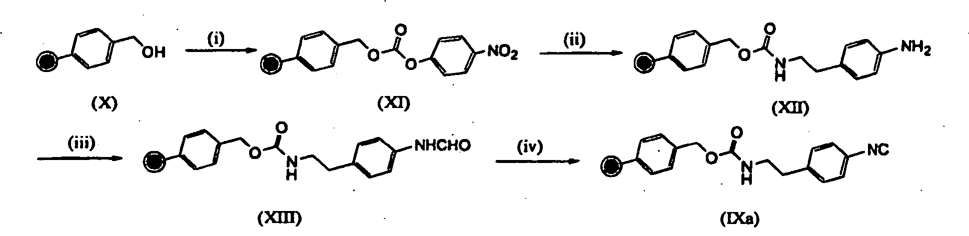

Reagents and Conditions:- (i) Wang resin, 4-nitrophenylchloroformate (5 equiv.), *n*-methyl morpholine (10 equiv.), THF. (ii) 2-(4 amino phenyl)ethylamine (5 equiv.), DMF. (iii) Formic acid (excess), acetic anhydride (excess), CH₂Cl₂ (iv) Ph₃P (5 equiv.), CCl₄ (5 equiv.), Et₃N (5 equiv.), CH₂Cl₂.

### Experimental Procedures

### Isonitrile Resin (IXa) :

The formamide resin (XIII) (50.0 g, 44.5 mmol) was swelled in anhydrous CH₂Cl₂ (500 ml). Triphenylphosphine (58.4 g, 222.5 mmol), carbon tetrachloride (21.5 ml, 222.5 mmol), and triethylamine (31.0 ml, 222.5 mmol) were added sequentially at RT. The reaction was mixed on an orbital shaker for 4.5 hours at RT. The reaction solution was drained off and the resin product (IXa) was washed with CH₂Cl₂ (20X), THF (10X), and Et₂O (10X). The resin was then placed in a vacuum oven at RT overnight to dry. IR analysis showed a sharp peak for the isonitrile at 2121 cm^{-1.}

### General methodology for the preparation of 1,4-Benzodiazepine-2,5-diones (I)

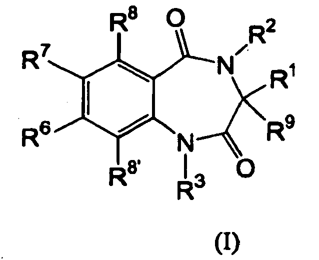

In general terms, compounds of formula (I) wherein R¹, R², R³, R^{8',} R⁶, R⁷, R⁸ and R⁹ are hereinbefore defined, may be synthesized by reacting a compound of formula (XIV) wherein R³, R^{8'}, R⁶, R⁷ and R⁸ are hereinbefore defined and Z¹ is a suitable amine protecting group, with a compound of formula (XV) wherein R¹ and R⁹ are hereinbefore defined, a compound of formula (XVI) wherein R² is hereinbefore defined, and a compound of formula (IX) wherein R¹² represents alkyl, aralkyl, aryl, fused arylcycloalkyl, fused arylheterocyclyl, cycloalkyl, heteroaralkyl, heteroaryl, fused heteroarylcycloalkyl, fused heteroarylheterocyclyl, heterocyclyl; in a suitable solvent at about room temperature, to afford the intermediate compound (XVII), wherein R¹, R², R³, R^{8',} R⁶, R⁷, R⁸, R⁹, R¹² and Z¹ are hereinbefore defined. The general reaction is illustrated in scheme 1 below:

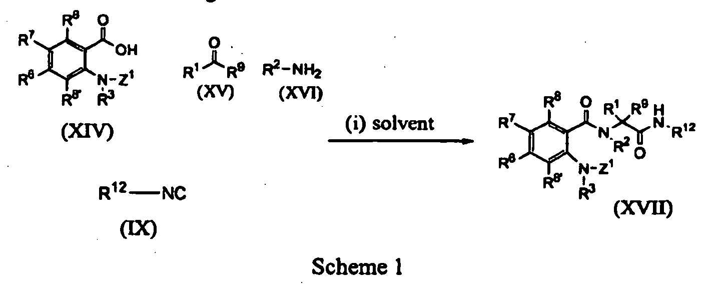

There is no particular restriction on the nature of the solvent to be employed, provided that it has no adverse effect on the reaction or on the reagents involved (See Waki et al. J. Am. Chem. Soc., 1977, 6075-6077). Examples of suitable solvents include: alcohols, such as methanol, 1-butanol, phenol, trifluoroethanol, hexafluoro-2-propanol; hydrocarbons, such as benzene and toluene; amides, such as dimethyl acetamide, dimethylformamide; halides, such as dichloromethane, dichloroethane; and ethers, such as tetrahydrofuran and dioxane; other solvents include water, 1-methyl-2-pyrrolidine, diethyl phosphite, tetramethylsulphone, dimethyl sulphoxide, acetonitrile and pyridine. Of these solvents, the alcohols are preferred.

There is no restriction on the isonitrile (IX) used in the reaction scheme 1 above, provided that it has no adverse effect on the reaction involved. Examples of suitable isonitriles include, 1-isocyanocyclohexene, benzyl isocyanide, n-butyl isocyanide, diethyl isocyanomethyl phosphonate, cyclohexyl isocyanide, 2,6-dimethylphenyl isocyanide, methyl isocyanoacetate, isopropyl isocyanide and 1,1,3,3-tetramethylbutyl isocyanide. Preferable isonitriles include the isonitrile functionalized polymer resin (IXa) or (XVIII), 1-isocyanocyclohexene (IXb), benzyl isocyanide, n-butyl isocyanide, diethyl isocyanomethyl phosphonate. More preferably, isonitriles used in the reaction are isonitrile functionalized polymer resin (IXa) 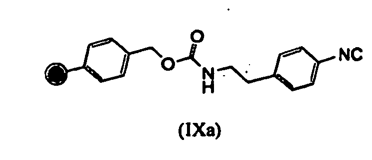 or the isonitrile functionalized polymer resin (XVIII) (A. Piscopio, ORG Poster 232, American Chemical Society Meeting, Las Vegas, NV, 7-10 Sept., 1997): 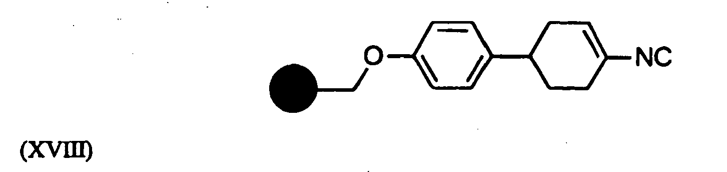 or 1-isocyanocyclohexene (IXb):

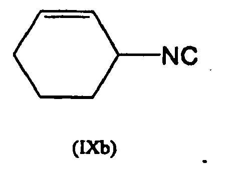

The use of resin bound isonitriles (IXa) or (XVIII) in the synthesis of compounds of the formula (I), (II), (III) or (V), is advantageous over other non-resin bound isonitriles. The use of the resin bound isonitriles allow excess amount of reagents to be used in the reaction to drive the Ugi reaction forward. Also, unlike solution phase procedures, these reagents can be easily removed by subsequent washing of the resin, leaving the Ugi product clean and resin bound

The reaction can take place over a wide range of temperatures, and the precise reaction temperature is not critical to the invention. In general, we find it convenient to carry out the reaction at a temperature of from about 0°C to about 150°C , preferably from room temperature to about 100°C, more preferably at about room temperature. The time required for the reaction may also vary widely, depending on many factors, notably the reaction temperature and the nature of the reagents. However, provided that the reaction is effected under the preferred conditions outlined above, a period of from 3 hours to 36 hours will usually suffice.

The intermediate compound of the formula (XVII) thus prepared may be recovered from the reaction mixture by conventional means. For example, the compounds may be recovered by distilling off the solvent in vacuo from the reaction mixture or, if necessary after distilling off the solvent from the reaction mixture, pouring the residue into water followed by extraction with a water-immiscible organic solvent and distilling off the solvent from the extract. Additionally, the product can, if desired, be further purified by various well techniques, such as recrystallization, reprecipitation or the various chromatography techniques, notably column chromatography or preparative thin layer chromatography. The intermediate compound is preferably recovered from the reaction mixture by distilling off the solvent in vacuo.

The intermediate compound (XVII) may be converted to a compound of formula (I) by reacting with acid and optionally base, in a suitable solvent and appropriate temperature, to effect removal of the amine protecting group (Z'), followed by cyclization. This reaction is illustrated in Scheme 2 .

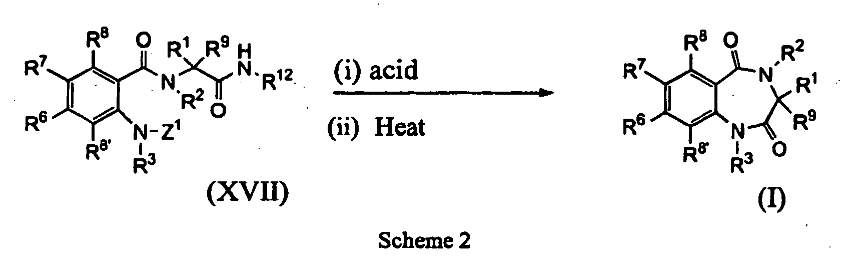

This reaction is carried out in the presence of an acid. There is no particular restriction on the nature of the acid to be used in this reaction, and any acid conventionally used to facilitate removal of an acid labile amine protecting group Z¹ and cyclization, may equally be used here, provided that it has no adverse effect on other parts of the molecule. Examples of suitable acids include: mineral acids such as hydrochloric acid or sulfuric acid; organic acids such as trifluoroacetic acid. Acids to be used in the reaction can also be generated in situ, for example by the addition of acetyl chloride in methanol, to generate hydrochloric acid. Preferably, anhydrous acids are used.

In addition to carrying out the reaction in scheme 2 in the presence of acid, a reaction step involving basic conditions can also be optionally carried out so as to facilitate the removal of the amine protecting group Z¹, wherein Z¹ is a base labile amine protecting group. There is no particular restriction on the nature of the base to be used in this reaction, and any base conventionally used to facilitate removal of a base labile amine protecting group Z¹, may equally be used here, provided that it has no adverse effect on other parts of the molecule. Examples of suitable bases include: organic bases such as ammonia, piperidine, morpholine, ethanolamine and diethylamine.

In situations where acidic conditions are used to remove the amine protecting group in (XVII) it may also be necessary to treat the deprotected intermediate, which is present as an acid salt, with base so as to convert the acid addition salt to its corresponding free base. There is no particular restriction on the nature of the base to be used. A base conventionally used to convert an acid addition salt to its corresponding free base form may be used here, provided that it has no adverse effect on other parts of the molecule. Examples of suitable bases include ammonia, piperidine, morpholine, ethanolamine, diethylamine, polystyrene bound di-isopropylethylamine or basic DOWEX. Preferably diethylamine, basic DOWEX or polystyrene bound di-isopropylethylamine.

This reaction can take place over a wide range of temperatures, and the precise reaction temperature is not critical to the invention. In general, we find it convenient to carry out the reaction at a temperature of from about 0°C to about 150°C , preferably from room temperature to about 100°C, more preferably at about room temperature. The time required for the reaction may also vary widely, depending on many factors, notably the reaction temperature and the nature of the reagents. However, provided that the reaction is effected under the preferred conditions outlined above, a period of from 3 hours to 36 hours will usually suffice.

There is no particular restriction on the amine protecting group (Z¹) employed. However, amino acid protecting groups which allow removal of the protecting group and cyclization of the deprotected intermediate, without purification or isolation of intermediates, are preferred. Examples of amine protecting groups include both acid labile amine protecting groups and base labile protecting groups. Preferred acid labile amine protecting group include tert-butoxycarbonyl (BOC) and 2-(4-biphenylyl)-isopropoxy carbonyl (BPOC). Preferred base labile amine protecting group include 9-fluoroenylmethyl carbamate (FMOC).

The use of the novel resin bound isonitrile (IXa) in the synthesis of compounds of the formula (I), wherein R¹² is the novel resin bound isonitrile derivative (IXa), is advantageous over other non-resin bound isonitriles. However, use of (IXa) in the synthesis of (I) involves activation of the resin linker to facilitate cleavage of the resin linkage and cyclization to afford (I). An illustration of the use of the novel resin bound isonitrile (IXa) in the synthesis of the intermediate formula (XIX) is illustrated in Scheme 3.

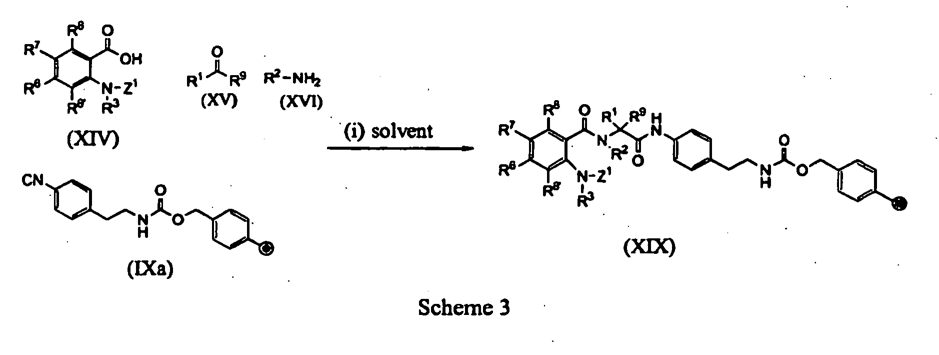

Activation of the benzamide carbonyl of (XIX) to give the intermediate (XX), wherein Z² is a carbamate protective group promotes facile cleavage from the resin. Examples of carbamate protective groups which may activate the benzamide group, provided that they no adverse effect on the reaction involved are, for example, t-butyl-O-CO- (BOC), benzyl-O-CO- (CBZ), Cl₃CCH₂-O-CO- (Troc), (CH₃)₃SiCH₂CH₂-O-CO- (TEOC), 1-methyl-1-(4-biphenylyl)ethyl-O-CO- (BPOC) and cycloalkyl-O-CO-. Other carbamate protective groups include those described in 'Protecting groups in Organic Synthesis' Greene, 1981, p. 223-49. An example of the activation of the benzamide group is illustrated in Scheme 4, wherein Z²X a suitable carbamate protective group reagent, for example (BOC)₂O. 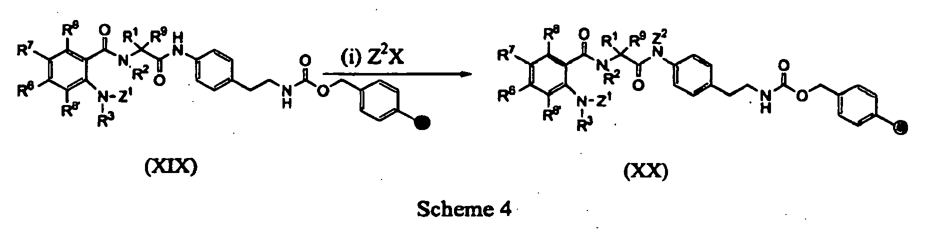 The reaction conditions and reagents used in the activation of the benzamide, are ones that are known in the art of for the conversion of an amine to a carbamate group. This sort of reaction is usually carried out in dichloromethane, in the presence of a base, for example Et₃N, and a catalytic amount of DMAP. (For other reaction conditions, see 'Protecting groups in Organic Synthesis' Greene, 1981, p. 223-49).

The resin "safety catch" linker is then cleaved, to facilitate the removal of the resin, by reacting the activated benzamide compound (XX) with an appropriate alkoxide, or hydroxide, to afford the corresponding alkyl ester or carboxylic acid derivative (XXI) respectively, wherein R¹³ is, for example, H, alkyl, phenyl or cycloalkyl (Mjalli, A.M.M., Sarshar, S., Baiga, T.J. Tetrahedron Lett. 1996, 37, 2943; Flynn, D. L., Zelle, R. E., Grieco, P. A. J. Org. Chem. 1983, 48, 2424.). An example of the cleavage from the resin is illustrated in reaction Scheme 5:

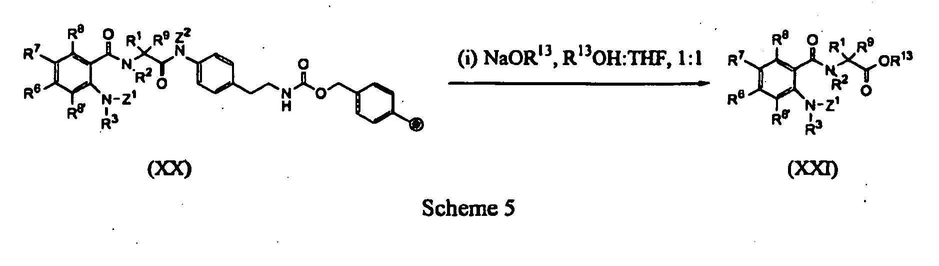

The intermediate compound (XXI) may be converted to a compound of formula (I) by reacting with acid, in a suitable solvent and appropriate temperature, to effect removal of the amine protecting group (Z¹), followed by cyclization. This reaction is illustrated in Scheme 6. 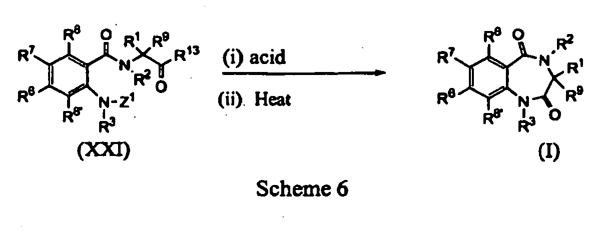 This reaction is carried out in the presence of an acid. There is no particular restriction on the nature of the acid to be used in this reaction, and any acid conventionally used to facilitate removal of an acid labile amine protecting group Z¹ and cyclization, may equally be used here, provided that it has no adverse effect on other parts of the molecule. Examples of suitable acids include: mineral acids such as hydrochloric acid or sulfuric acid; organic acids such as trifluoroacetic acid. Acids to be used in the reaction can also be generated in situ, for example by the addition of acetyl chloride in methanol, to generate hydrochloric acid. Preferably, anhydrous acids are used.

In addition to carrying out the reaction in scheme 6 in the presence of acid, a reaction step involving basic conditions can also be optionally carried out so as to facilitate the removal of the amine protecting group Z¹, wherein Z¹ is a base labile amine protecting group. There is no particular restriction on the nature of the base to be used in this reaction, and any base conventionally used to facilitate removal of an base labile amine protecting group Z¹, may equally be used here, provided that it has no adverse effect on other parts of the molecule. Examples of suitable bases include: organic bases such as ammonia, piperidine, morpholine, ethanolamine and diethylamine.

This reaction can take place over a wide range of temperatures, and the precise reaction temperature is not critical to the invention. In general, we find it convenient to carry out the reaction at a temperature of from about 0°C to about 150°C , preferably from room temperature to about 100°C, more preferably at about room temperature. The time required for the reaction may also vary widely, depending on many factors, notably the reaction temperature and the nature of the reagents. However, provided that the reaction is effected under the preferred conditions outlined above, a period of from 3 hours to 36 hours will usually suffice.

There is no particular restriction on the amine protecting group (Z¹) employed. However, amino acid protecting groups which allow removal of the protecting group and cyclization of the deprotected intermediate, without purification or isolation of intermediates, are preferred. Examples of amine protecting groups include both acid labile amine protecting groups and base labile protecting groups. Preferred acid labile amine protecting group include tert-butoxycarbonyl (BOC) and 2-(4-biphenylyl)-isopropoxy carbonyl (BPOC). Preferred base labile amine protecting group include 9-fluoroenylmethyl carbamate (FMOC).

In situations where acidic conditions are used to remove the amine protecting group in (XXI) it may also be necessary to treat the deprotected intermediate, which is present as an acid salt, with base so as to convert the acid addition salt to its corresponding free base. There is no particular restriction on the nature of the base to be used. A base conventionally used to convert an acid addition salt to its corresponding free base form may be used here, provided that it has no adverse effect on other parts of the molecule. Examples of suitable bases include ammonia, piperidine, morpholine, ethanolamine, diethylamine, polystyrene bound di-isopropylethylamine or basic DOWEX. Preferably diethylamine, basic DOWEX or polystyrene bound di-isopropylethylamine.

Similarly, solid phase synthesis of a compound of formula (I) can be carried out using the resin bound isonitrile of formula (XVIII) using reaction conditions similar to those described for scheme 1 and scheme 2.

According to a further feature of the present invention, compounds may be prepared by interconversion of other compounds.

A compound of formula (I)including a group containing one or more nitrogen ring atoms, preferably imine (=N-), may be converted to the corresponding compound wherein one or more nitrogen ring atom of the group is oxidized to an N-oxide, preferably by reacting with a peracid, for example peracetic acid in acetic acid or m-chloroperoxybenzoic acid in an inert solvent such as dichloromethane, at a temperature from about room temperature to reflux, preferably at elevated temperature.

As an example of the interconversion process, compounds (I) containing sulphoxide linkages may be prepared by the oxidation of corresponding compounds containing -S-linkages. For example, the oxidation may conveniently be carried out by means of reaction with a peroxyacid, e.g. 3-chloroperbenzoic acid, preferably in an inert solvent, e.g. dichloromethane, preferably at or near room temperature, or alternatively by means of potassium hydrogen peroxomonosulphate in a medium such as aqueous methanol, buffered to about pH 5, at temperatures between about 0°C and room temperature. This latter method is preferred for compounds containing an acid-labile group.

As another example of the interconversion process, compounds (I) containing sulphone linkages may be prepared by the oxidation of corresponding compounds containing -S- or sulphoxide linkages. For example, the oxidation may conveniently be carried out by means of reaction with a peroxyacid, e.g. 3-chloroperbenzoic acid, preferably in an inert solvent, e.g. dichloromethane, preferably at or near room temperature.

It will be understood that designation of aromaticity with respect to carbocycles and heterocycles herein includes any highly resonant unsaturated ring structure. Alternatively, placement of double bonds, where indicated, represents one potential structure for the depicted compound but will be understood to include other resonant states of the compound as well as protonated and charged species, only one of which may be shown.

It will be appreciated that compounds may contain asymmetric centers. These symmetric centers may independently be in either the R or S configuration. It will be apparent to those skilled in the art that certain compounds may also exhibit geometrical isomerism. It is to be understood that the compounds include individual geometrical isomers and stereoisomers and mixtures thereof, including racemic mixtures, of compounds of formula (I) hereinabove. Such isomers can be separated from their mixtures, by the application or adaptation of known methods, for example chromatographic techniques and recrystallization techniques, or they are separately prepared from the appropriate isomers of their intermediates.

For the purpose herein it is understood that tautomeric forms are included in the recitation of a given group, e.g., thio/mercapto or oxo/hydroxyl.

Acid additional salts are formed with the compounds in which a basic function such as an amino, alkylamino, or dialkylamino group is present. The pharmaceutically acceptable, i.e., nontoxic, acid addition salts are preferred. The salts chosen are chosen optimally to be compatible with the customary pharmaceutical vehicles and adapted for oral or parenteral administration. Acid addition salts of the compounds may be prepared by reaction of the free base with the appropriate acid, by the application or adaptation of known methods. For example, the acid addition salts of the compounds may be prepared either by dissolving the free base in water or aqueous alcohol solution or other suitable solvents containing the appropriate acid and isolating the salt by evaporating the solution, or by reacting the free base and acid in an organic solvent, in which case the salt separates directly or can be obtained by concentration of the solution. Some suitable acids for use in the preparation of such salts are hydrochloric acid, hydrobromic acid, phosphoric acid, sulfuric acid, various organic carboxylic and sulfonic acids, such as acetic acid, citric acid, propionic acid, succinic acid, benzoic acid, tartaric acid, fumaric acid, mandelic acid, ascorbic acid, malic acid, methanesulfonic acid, toluenesulfonic acid, fatty acids, adipate, alginate, ascorbate, aspartate, benzenesulfonate, benzoate, cyclopentanepropionate, digluconate, dodecylsulfate, bisulfate, butyrate, lactate, laurate, lauryl sulfate, malate, hydroiodide, 2-hydroxy-ethanesulfonate, glycerophosphate, picrate, pivalate, pamoate, pectinate, persulfate, 3-phenylpropionate, thiocyanate, 2-naphthalenesulfonate, undecanoate, nicotinate, hemisulfate, heptonate, hexanoate, camphorate, camphersulfonate, and others.

The acid addition salts of the compounds can be regenerated from the salts by the application or adaptation of known methods. For example, parent compounds can be regenerated from their acid addition salts by treatment with an alkali, e.g. aqueous sodium bicarbonate solution or aqueous ammonia solution.

Compounds can be regenerated from their base addition salts by the application or adaptation of known methods. For example, parent compounds can be regenerated from their base addition salts by treatment with an acid, e.g. hydrochloric acid.

Base addition salts may be formed where the compound contains a carboxy group, or a sufficiently acidic bioisostere. The bases which can be used to prepare the base addition salts include preferably those which produce, when combined with the free acid, pharmaceutically acceptable salts, that is, salts whose cations are non-toxic to the patient in pharmaceutical doses of the salts, so that the beneficial inhibitory effects inherent in the free base are not vitiated by side effects ascribable to the cations. Pharmaceutically acceptable salts, including those derived from alkali and alkaline earth metal salts, within the scope of the invention include those derived from the following bases: sodium hydride, sodium hydroxide, potassium hydroxide, calcium hydroxide, aluminium hydroxide, lithium hydroxide, magnesium hydroxide, zinc hydroxide, ammonia, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)-aminomethane, tetramethylammonium hydroxide, and the like.

Compounds may be conveniently prepared, or formed during the process of the invention, as solvates (e.g. hydrates). Hydrates of compounds may be conveniently prepared by recrystallization from an aqueous/organic solvent mixture, using organic solvents such as dioxan, tetrahydrofuran or methanol.

### EXAMPLE 1

### General Solid Phase Synthesis of Compounds of Formula (I) using the Ugi reaction and resin (IXa)

(60mg) of resin (IXa) is pre-swelled with THF. 0.5M solutions of the aldehyde (XV) (10 equiv.), amine (XVI) (10 equiv.) and carboxylic acid (XIV) (10 equiv.) in THF:MeOH (1:1) are added sequentially to the resin (IXa) and the reaction stirred at room temperature for 3 days. The resin is washed sequentially with CH₂Cl₂, THF, DMF, THF and MeOH dried under high vacuum to yield the resin bound Ugi product (IXa) Treatment with BOC₂O (10 equiv.), Et₃N (10 equiv.) and DMAP in CH₂Cl₂ (15 hours) afforded the activated resin (XX) for cleavage. Sodium methoxide (5mg) in THF: MeOH, 1:1, is added to the resin and shaken for 20 hours. The solvent is evaporated in vacuo to give the desired methyl ester (XXI). The deprotection/cyclization steps are performed using either a 10% solution of acetyl chloride in methanol, or a 10% solution of trifluoroacetic acid in dichloroethane. The samples are then evaporated in a SAVANT at room temperature for 3 hours to give the crude product of formula (I). Examples of products (examples 23 to 28) synthesized using this general methodology are indicated below and purities are determined by lc/ms (liquid chromatography/mass spectrometry) ELSD (evaporative light scattering detector) A% and UV A%. Lc/ms analysis is performed using a Hypersil BDS 3 m C18 4.6X50mm 0.1%TFA in H₂O/CH₃N 10% to 100% CH₃N 5 min, at a rate of 1ml/min for **23-28.** Desired products are seen as (M+ 1).

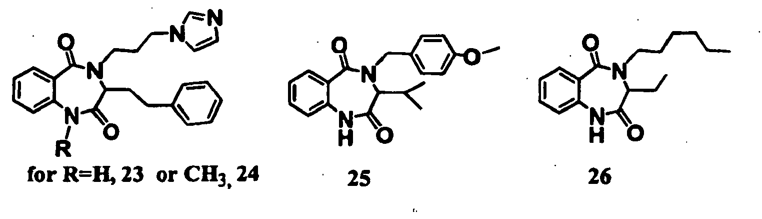

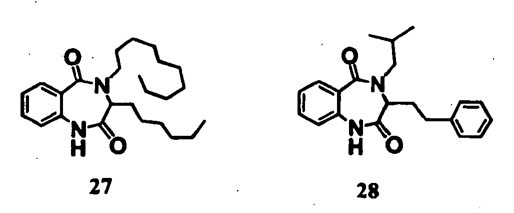

| Compound | Retention time | mass spec | ELSD A% | UV (220 nm) A% |
|---|---|---|---|---|
| 23 | 3.36 | 388 | >90 | >90 |
| 24 | 3.62 | 402 | 90 | >90 |
| 25 | 3.93 | 338 | 90 | 80 |
| 26 | 4.27 | 288 | 90 | 91 |
| 27 | 6.48 | 400 | 95 | 91 |
| 28 | 4.36 | 336 | 89 | 80 |

## Claims

1. A method for preparing a N-[(aliphatic or aromatic)carbonyl)]-2-aminoacetamide compound of the formula 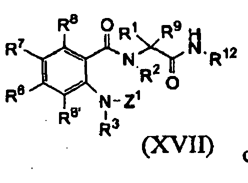 comprising
reacting the following four compounds:
a carbonyl compound of formula 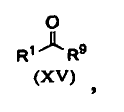
an amine compound of formula
R²-NH₂ (XVI),
an isonitrile compound of formula
R¹²-NC (IX),
and
an acid compound of formula 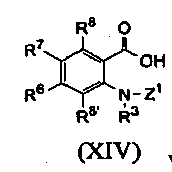 wherein Z¹ is a suitable amine protecting group,
to produce the N-[(aliphatic or aromatic)carbonyl)]-2-aminoacetamide compound;
wherein:
R¹ and R⁹ independently are selected from hydrogen, alkenyl, alkyl, aralkenyl, aralkyl, aryl, fused arylcycloalkenyl, fused arylcycloalkyl, fused arylheterocyclenyl, fused arylheterocyclyl, cycloalkyl, cycloalkenyl, heteroaralkenyl, heteroaralkyl, heteroaryl, fused heteroarylcycloalkenyl, fused heteroarylcycloalkyl, fused heteroarylheterocyclenyl, fused heteroarylheterocyclyl, heterocyclenyl, and heterocyclyl;
R² is selected from hydrogen, alkenyl, alkyl, aralkyl, aryl, fused arylcycloalkenyl, fused arylcycloalkyl, fused arylheterocyclenyl, fused arylheterocyclyl, cycloalkyl, cycloalkenyl, heteroaralkyl, heteroaryl, fused heteroarylcycloalkenyl, fused heteroarylcycloalkyl, fused heteroarylheterocyclenyl, fused heteroarylheterocyclyl, heterocyclenyl and heterocyclyl;
R³ is selected from hydrogen, alkenyl, alkyl, aralkyl, aryl, fused arylcycloalkenyl, fused arylcycloalkyl, fused arylheterocyclenyl, fused arylheterocyclyl, cycloalkyl, cycloalkenyl, heteroaralkyl, heteroaryl, fused heteroarylcycloalkenyl, fused heteroarylcycloalkyl, fused heteroarylheterocyclenyl, fused heteroarylheterocyclyl, heterocyclenyl and heterocyclyl;
R⁶, R⁷, R⁸ and R^{8'} independently are selected from hydrogen, alkenyl, alkenyloxy, alkoxy, alkyl, aryl, alkylsulfinylcarbamoyl, alkynyl, alkynyloxy, aralkenyl, aralkylsulfonyl, aralkynyl, fused arylcycloalkenyl, fused arylcycloalkyl, fused arylheterocyclenyl, fused arylheterocyclyl, aryloxycarbonyl, cycloalkyloxy, heteroaralkenyl, heteroaralkyloxy, heteroaralkynyl, heteroaroyl, fused heteroarylcycloalkenyl, fused heteroarylcycloalkyl, fused heteroarylheterocyclenyl, fused heteroarylheterocyclyl, heteroarylsulphonylcarbamoyl, heterocyclyloxy, heteroaryl, aralkyl, heteroaralkyl, hydroxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, aryldiazo, heteroaryldiazo, amidino, Y¹Y²N-, Y¹Y²NCO- and Y¹Y²NSO₂-, wherein Y¹ and Y² are independently hydrogen, alkyl, aryl, aralkyl or heteroaralkyl, or where the substituent is Y¹Y²N-, then one of Y¹ and Y² may be acyl or aroyl and the other of Y¹ and Y² is as defined previously, or where the substituent is Y¹Y²NCO- or Y¹Y²NSO₂-, Y¹ and Y², taken together with the N atom through which Y¹ and Y² are linked, may also form a 4 to 7 membered heterocyclyl or heterocyclenyl, or
R³ and R^{8'} , taken together with the nitrogen atom and carbon atoms through which R³ and R^{8'} are linked, form a 5 to 7 membered heterocyclyl or heterocyclenyl, or
two adjacent substituents selected from the substituents R⁶, R⁷, R^{8'} and R⁸, taken together with the aryl carbon atoms through which the two adjacent substituents are linked, form a 5 to 7 membered cycloalkyl or a cycloalkenyl, heterocyclyl or heterocyclenyl, or 6 membered aryl or 5 or 6 membered heteroaryl ring; and
wherein
the isonitrile compound (IX) is selected from the group of formulae 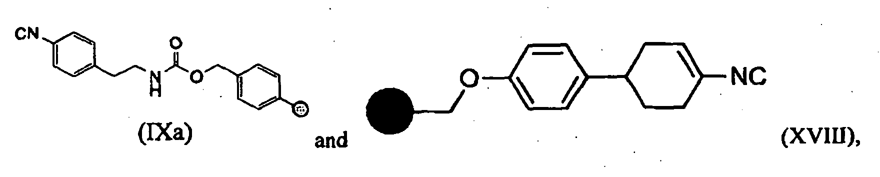 where 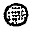 is a solid support resin.

2. The method according to claim 1 further comprising deprotecting and cyclizing the N-[(aliphatic or aromatic)carbonyl)]-2-aminoacetamide compound of formula (A) to afford a cyclized compound having a formula:

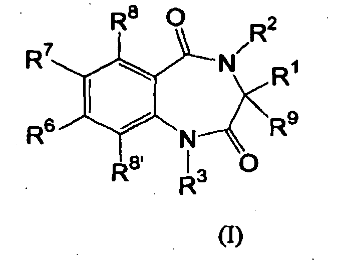

3. The method according to claim 2 wherein the isonitrile compound is (IXa), the N-[(aliphatic or aromatic)carbonyl)]-2-aminoacetamide compound is of the formula 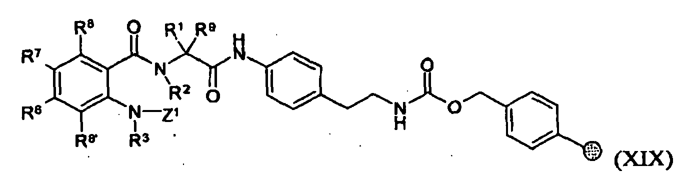 and the method comprises the steps of activating the benzamide carbonyl of (XIX) with a carbamate protecting group, and reacting with an alkoxide or hydroxide to cleave from the resin and afford a compound of the formula 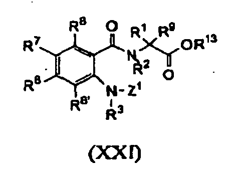 wherein R¹³ is H, alkyl, phenyl or cycloalkyl, followed by cyclization.

4. The method according to claim 2 wherein the cyclized product is selected from the group consisting of:

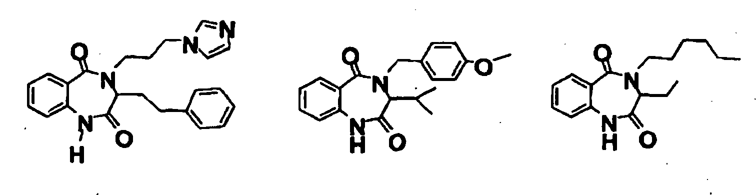

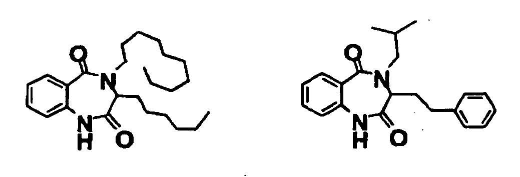

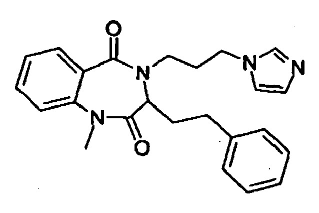

5. A resin bound isonitrile of formula 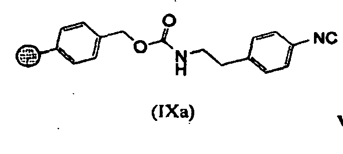 wherein 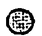 is a solid support resin.

## Patentansprüche

1. Verfahren zur Herstellung einer N-[(aliphatisch oder aromatisch)Carbonyl]-2-aminoacetamidverbindung der Formel 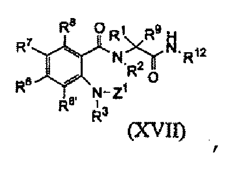 bei dem man
die folgenden vier Verbindungen:
eine Carbonylverbindung der Formel 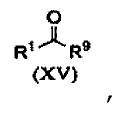
eine Aminverbindung der Formel
**R²-NH₂** **(XVI),**
eine Isonitrilverbindung der Formel
**R¹²-NC** **(IX)**
und
eine Säureverbindung der Formel 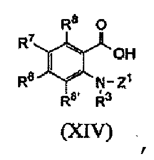 wobei Z¹ für eine geeignete Aminschutzgruppe steht, unter Bildung der N-[(aliphatisch oder aromatisch)Carbonyl]-2-aminoacetamidverbindung umsetzt;
wobei:
R¹ und R⁹ unabhängig voneinander ausgewählt sind aus Wasserstoff, Alkenyl, Alkyl, Aralkenyl, Aralkyl, Aryl, kondensiertem Arylcycloalkenyl, kondensiertem Arylcycloalkyl, kondensiertem Arylheterocyclenyl, kondensiertem Arylheterocyclyl, Cycloalkyl, Cycloalkenyl, Heteroaralkenyl, Heteroaralkyl, Heteroaryl, kondensiertem Heteroarylcycloalkenyl, kondensiertem Heteroarylcycloalkyl, kondensiertem Heteroarylheterocyclenyl, kondensiertem Heteroarylheterocyclyl, Heterocyclenyl und Heterocyclyl;
R² ausgewählt ist aus Wasserstoff, Alkenyl, Alkyl, Aralkyl, Aryl, kondensiertem Arylcycloalkenyl, kondensiertem Arylcycloalkyl, kondensiertem Arylheterocyclenyl, kondensiertem Arylheterocyclyl, Cycloalkyl, Cycloalkenyl, Heteroaralkyl, Heteroaryl, kondensiertem Heteroarylcycloalkenyl, kondensiertem Heteroarylcycloalkyl, kondensiertem Heteroarylheterocyclenyl, kondensiertem Heteroarylheterocyclyl, Heterocyclenyl und Heterocyclyl;
R³ ausgewählt ist aus Wasserstoff, Alkenyl, Alkyl, Aralkyl, Aryl, kondensiertem Arylcycloalkenyl, kondensiertem Arylcycloalkyl, kondensiertem Arylheterocyclenyl, kondensiertem Arylheterocyclyl, Cycloalkyl, Cycloalkenyl, Heteroaralkyl, Heteroaryl, kondensiertem Heteroarylcycloalkenyl, kondensiertem Heteroarylcyclalkyl, kondensiertem Heteroarylheterocyclenyl, kondensiertem Heteroarylheterocyclyl, Heterocyclenyl und Heterocyclyl;
R⁶, R⁷, R⁸ und R^{8'} unabhängig voneinander ausgewählt sind aus Wasserstoff, Alkenyl, Alkenyloxy, Alkoxy, Alkyl, Aryl, Alkylsulfinylcarbamoyl, Alkinyl, Alkinyloxy, Aralkenyl, Aralkylsulfonyl, Aralkinyl, kondensiertem Arylcycloalkenyl, kondensiertem Arylcycloalkyl, kondensiertem Arylheterocyclenyl, kondensiertem Arylheterocyclyl, Aryloxycarbonyl, Cycloalkyloxy, Heteroaralkenyl, Heteroaralkyloxy, Heteroaralkinyl, Heteroaroyl, kondensiertem Heteroarylcycloalkenyl, kondensiertem Heteroarylcycloalkyl, kondensiertem Heteroarylheterocyclenyl, kondensiertem Heteroarylheterocyclyl, Heteroarylsulfonylcarbamoyl, Heterocyclyloxy, Heteroaryl, Aralkyl, Heteroaralkyl, Hydroxy, Aryloxy, Aralkoxy, Acyl, Aroyl, Halogen, Nitro, Cyano, Carboxy, Alkoxycarbonyl, Aryloxycarbonyl, Aralkoxycarbonyl, Alkylsulfonyl, Arylsulfonyl, Heteroarylsulfonyl, Alkylsulfinyl, Arylsulfinyl, Heteroarylsulfinyl, Alkylthio, Arylthio, Heteroarylthio, Aralkylthio, Heteroaralkylthio, Cycloalkyl, Cycloalkenyl, Heterocyclyl, Heterocyclenyl, Aryldiazo, Heteroaryldiazo, Amidino, Y¹Y²N-, Y¹Y²NCO- und Y¹Y²NSO₂-, wobei Y¹ und Y² unabhängig voneinander für Wasserstoff, Alkyl, Aryl, Aralkyl oder Heteroaralkyl stehen, oder, wenn es sich bei dem Substituenten um Y¹Y²N- handelt, einer der Reste Y¹ und Y² für Acyl oder Aroyl stehen kann und der andere der Reste Y¹ und Y² wie oben definiert ist, oder, wenn es sich bei dem Substituenten um Y¹Y²NCO- oder Y¹Y²NSO₂- handelt, Y¹ und Y² zusammen mit dem N-Atom, über das Y¹ und Y² miteinander verbunden sind, auch ein 4- bis 7-gliedriges Heterocyclyl oder Heterocyclenyl bilden können, oder
R³ und R^{8'} zusammen mit dem Stickstoffatom und den Kohlenstoffatomen, über die R³ und R^{8'} miteinander verbunden sind, ein 5- bis 7-gliedriges Heterocyclyl oder Heterocyclenyl bilden, oder
zwei benachbarte, unter den Substituenten R⁶, R⁷, R^{8'} und R⁸ ausgewählten Substituenten zusammen mit den Arylkohlenstoffatomen, über die die beiden benachbarten Substituenten miteinander verbunden sind, ein 5- bis 7-gliedriges Cycloalkyl oder ein Cycloalkenyl, Heterocyclyl oder Heterocyclenyl, oder einen 6-gliedrigen Arylring oder einen 5- oder 6-gliedrigen Heteroarylring bilden; und
wobei
die Isonitrilverbindung (IX) ausgewählt ist aus der Gruppe der Formeln 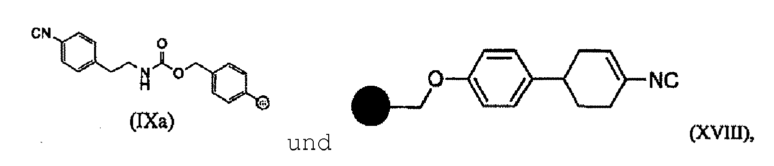 wobei 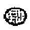 für ein festes Trägerharz steht.

2. Verfahren nach Anspruch 1, bei dem man weiterhin die N-[(aliphatisch oder aromatisch)Carbonyl]-2-aminoacetamidverbindung der Formel (A) unter Bildung einer cyclisierten Verbindung mit der Formel: 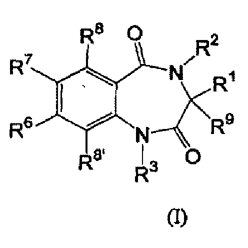 entschützt und cyclisiert.

3. Verfahren nach Anspruch 2, wobei es sich bei der Isonitrilverbindung um (IXa) handelt, es sich bei der N-[(aliphatisch oder aromatisch)Carbonyl]-2-aminoacetamidverbindung um die Formel 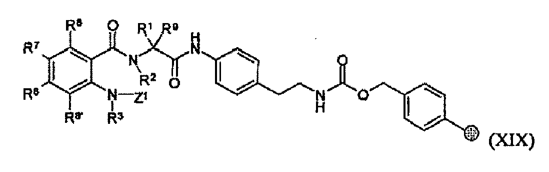 handelt und man bei dem Verfahren die Benzamidcarbonylgruppe von (XIX) mit einer Carbamatschutzgruppe aktiviert und zur Abspaltung vom Harz mit einem Alkoxid oder Hydroxid umsetzt, wodurch man eine Verbindung der Formel 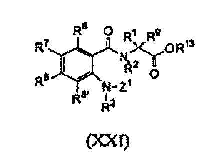 erhält, in welcher R¹³ für H, Alkyl, Phenyl oder Cycloalkyl steht, die anschließend cyclisiert wird.

4. Verfahren nach Anspruch 2, wobei das cyclisierte Produkt ausgewählt ist aus der Gruppe bestehend aus:

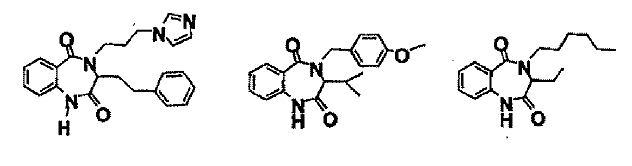

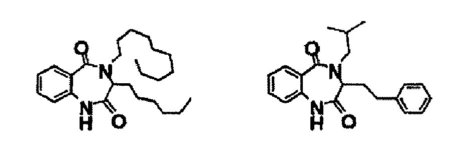

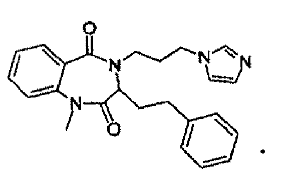

5. Harzgebundenes Isonitril der Formel 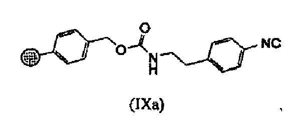 wobei 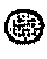 für ein festes Trägerharz steht.

## Revendications

1. Méthode de préparation d'un composé de N-[(carbonyl(aliphatique ou aromatique)]-2-aminoacétamide de formule 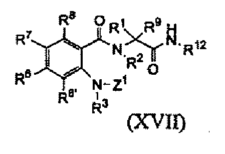 comprenant
la réaction des quatre composés suivantes :
un composé carbonyle de formule 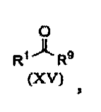
un composé amine de formule
**R²-NH₂** **(XVI),**
un composé isonitrile de formule
**R¹²-NC** **(IX),**
et
un composé acide de formule 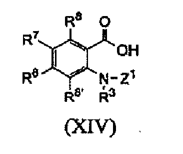 dans laquelle Z¹ est un groupement protecteur d'amine convenable,
pour produire le composé de N-[(carbonyl(aliphatique ou aromatique)]-2-aminoacétamide ;
dans lequel :
R¹ et R⁹ sont choisis indépendamment parmi hydrogène, alcényle, alkyle, aralcényle, aralkyle, aryle, arylcycloalcényle condensé, arylcycloalkyle condensé, arylhétérocyclényle condensé, arylhétérocyclyle condensé, cycloalkyle, cycloalcényle, hétéroaralcényle, hétéroaralkyle, hétéroaryle, hétéroarylcycloalcényle condensé, hétéroarylcycloalkyle condensé, hétéroarylhétérocyclényle condensé, hétéroarylhétérocyclyle condensé, hétérocyclényle et hétérocyclyle ;
R² est choisi parmi hydrogène, alcényle, alkyle, aralkyle, aryle, arylcycloalcényle condensé, arylcycloalkyle condensé, arylhétérocyclényle condensé, arylhétérocyclyle condensé, cycloalkyle, cycloalcényle, hétéroaralkyle, hétéroaryle, hétéroarylcycloalcényle condensé, hétéroarylcycloalkyle condensé, hétéroarylhétérocyclényle condensé, hétéroarylhétérocyclyle condensé, hétérocyclényle et hétérocyclyle ;
R³ est choisi parmi hydrogène, alcényle, alkyle, aralkyle, aryle, arylcycloalcényle condensé, arylcycloalkyle condensé, arylhétérocyclényle condensé, arylhétérocyclyle condensé, cycloalkyle, cycloalcényle, hétéroaralkyle, hétéroaryle, hétéroarylcycloalcényle condensé, hétéroarylcycloalkyle condensé, hétéroarylhétérocyclényle condensé, hétéroarylhétérocyclyle condensé, hétérocyclényle et hétérocyclyle ;
R⁶, R⁷, R⁸ et R^{8'} sont choisis indépendamment parmi hydrogène, alcényle, alcényloxy, alcoxy, alkyle, aryle, alkylsulfinylcarbamoyle, alcynyle, alcynyloxy, aralcényle, aralkylsulfonyle, aralcynyle, arylcycloalcényle condensé, arylcycloalkyle condensé, arylhétérocyclényle condensé, arylhétérocyclyle condensé, aryloxycarbonyle, cycloalkyloxy, hétéroalcényle, hétéroaralkyloxy, hétéroalcynyle, hétéroaroyle, hétéroarylcycloalcényle condensé, hétéroarylcycloalkyle condensé, hétéroarylhétérocyclényle condensé, hétéroarylhétérocyclyle condensé, hétéroarylsulfonylcarbamoyle, hétérocyclyloxy, hétéroaryle, aralkyle, hétéroaralkyle, hydroxy, aryloxy, aralcoxy, acyle, aroyle, halogéno, nitro, cyano, carboxy, alcoxycarbonyle, aryloxycarbonyle, aralcoxycarbonyle, alkylsulfonyle, arylsulfonyle, hétéroarylsulfonyle, alkylsulfinyle, arylsulfinyle, hétéroarylsulfinyle, alkylthio, arylthio, hétéroarylthio, aralkylthio, hétéroaralkylthio, cycloalkyle, cycloalcényle, hétérocyclyle, hétérocyclényle, aryldiazo, hétéroaryldiazo, amidino, Y¹Y²N-, Y¹Y²NCO- et Y¹Y²NSO₂- où Y¹ et Y² sont indépendamment hydrogène, alkyl, aryle, aralkyle ou hétéroaralkyle, ou où le substituant est Y¹Y²N-, alors l'un de Y¹ et Y² peut être acyle ou aroyle et l'autre de Y¹ et Y² est tel que défini précédemment, ou où le substituant est Y¹Y²NCO- ou Y¹Y²NSO₂-, Y¹ et Y², pris ensemble avec l'atome de N par l'intermédiaire duquel Y¹ et Y² sont liés, peuvent également former un hétérocyclyle ou hétérocyclényle à 4 à 7 chaînons, ou
R³ et R^{8'}, pris ensemble avec l'atome d'azote et les atomes de carbone par l'intermédiaire desquels R³ et R^{8'} sont liés, forment un hétérocyclyle ou hétérocyclényle à 5 à 7 chaînons, ou
deux substituants adjacents choisis parmi les substituants R⁶, R⁷, R^{8'} et R⁸, pris ensemble avec les atomes de carbone de l'aryle par l'intermédiaire desquels les deux substituants adjacents sont liés, forment un cycloalkyle à 5 à 7 chaînons ou un cycloalcényle, hétérocyclyle ou hétérocyclényle, ou un cycle aryle à 6 chaînons ou hétéroaryle à 5 ou 6 chaînons ; et
où
le composé isonitrile (IX) est choisi dans le groupe de formules 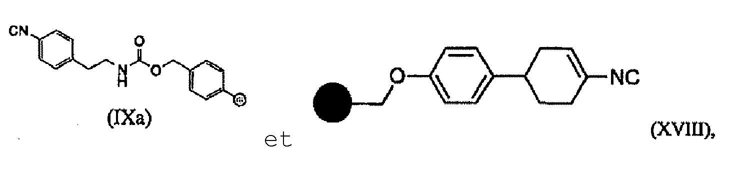 où 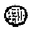 est une résine de support solide.

2. Méthode selon la revendication 1, comprenant en outre la déprotection et la cyclisation du composé de N-[(carbonyl(aliphatique ou aromatique)]-2-aminoacétamide de formule (A) pour donner un composé cyclisé ayant une formule :

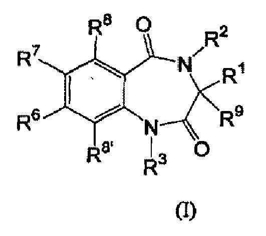

3. Méthode selon la revendication 2, **caractérisée en ce que** le composé isonitrile est (IXa), le composé de N-[(carbonyl(aliphatique ou aromatique)]-2-aminoacétamide est de formule 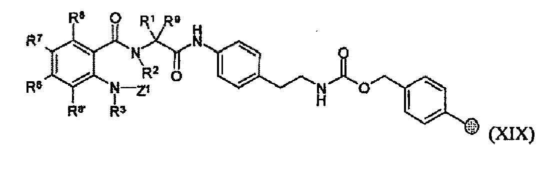 et la méthode comprend les étapes d'activation du benzamide carbonyle de (XIX) avec un groupement protecteur de carbamate, et de réaction avec un alcoxyde ou hydroxyde pour cliver de la résine pour donner un composé de formule 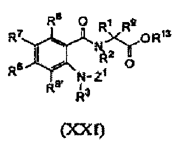 dans laquelle R¹³ est H, alkyle, phényle ou cycloalkyle, suivies d'une étape de cyclisation.

4. Méthode selon la revendication 2, **caractérisée en ce que** le produit cyclisé est choisi dans le groupe constitué de :

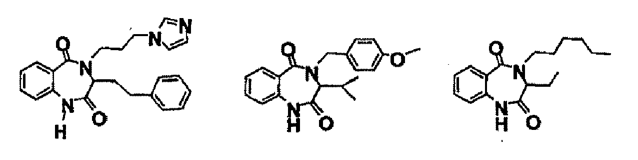

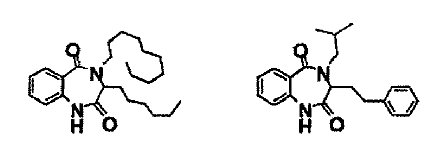

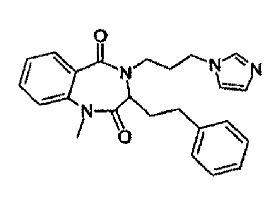

5. Isonitrile lié à une résine, de formule 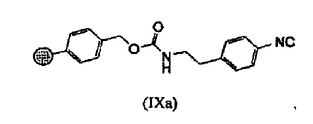 où 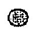 est une résine de support solide.
